(19) Europäisches Patentamt
European Patent Office
Office européen des brevets

(11) EP 0 917 465 B1

(12) # FASCICULE DE BREVET EUROPEEN

(45) Date de publication et mention de la délivrance du brevet:
**20.11.2002 Bulletin 2002/47**

(21) Numéro de dépôt: **97934589.9**

(22) Date de dépôt: **17.07.1997**

(51) Int Cl.[7]: **A61K 31/14**, A61K 31/40, A61K 31/445, C07D 295/02, A61P 33/02

(86) Numéro de dépôt international:
**PCT/FR97/01336**

(87) Numéro de publication internationale:
**WO 98/004252 (05.02.1998 Gazette 1998/05)**

(54) **AGENTS ANTIPALUDEENS ET ANTIBABESIOSES ET COMPOSITIONS PHARMACEUTIQUES LES CONTENANT**

MITTEL GEGEN MALARIA UND BABESIA UND DIESE ENTHALTENDE PFARMAZEUTISCHE ZUBEREITUNGEN

ANTIMALARIAL AND ANTI-BABESIOSIS AGENTS AND PHARMACEUTICAL COMPOSITIONS CONTAINING THE SAME AGENTS

(84) Etats contractants désignés:
**AT BE CH DE DK ES FI FR GB GR IE IT LI LU MC NL PT SE**

(30) Priorité: **31.07.1996 FR 9609678**

(43) Date de publication de la demande:
**26.05.1999 Bulletin 1999/21**

(73) Titulaire: **Virbac S.A.**
**06517 Carros (FR)**

(72) Inventeurs:
- **VIAL, Henri**
  **F-34080 Montpellier (FR)**
- **CALAS, Michèle**
  **F-34090 Montpellier (FR)**
- **ANCELIN, Marie-Laure**
  **F-34270 Saint Jean de Cuculles (FR)**
- **GIRAL, Louis**
  **F-34000 Montpellier (FR)**

(74) Mandataire: **Desaix, Anne et al**
**Ernest Gutmann - Yves Plasseraud S.A.**
**3, rue Chauveau-Lagarde**
**75008 Paris (FR)**

(56) Documents cités:
**WO-A-96/11910**

- **M. L. ANCELIN ET AL.: "Inhibitors of choline transport into Plasmodium-infected erythrocytes are effective antiplasmodial compounds in vitro" BIOCHEMICAL PHARMACOLOGY, vol. 34, no. 22, 1985, pages 4068-71, XP000646040**
- **M. L. ANCELIN ET AL.: "Quaternary Ammonium Compounds efficiently inhibit Plasmodium falciparum growth in vitro by impairment of choline transport" ANTIMICROBIAL AGENTS AND CHEMOTHERAPY, vol. 29, no. 5, 1986, pages 814-20, XP000646037**
- **J. T. HOLDEN ET AL.: "Inhibition of high-affinity choline transport in rat striatal synaptosomes by alkyl biquaternary ammonium compounds" MOLECULAR PHARMACOLOGY, vol. 11, no. 1, 1975, pages 19-27, XP000646446**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 917 465 B1

## Description

L'invention concerne des compositions pharmaceutiques ayant une activité antipaludéenne et antibabesiose ainsi que les principes actifs de ces compositions et leur utilisation dans l'obtention de médicaments antipaludéens.

Le paludisme (ou malaria ) reste la plus importante des maladies parasitaires qui sévit dans les zones intertropicales. Véritable composante de l'environnement, il constitue l'un des freins les plus puissant au développement de régions immenses. Tout habitant y est infecté, de sa naissance à sa mort, plusieurs fois par an, (jusqu'à 1 000 fois au Congo) et ne peut survivre que grâce à la prémunition qu'il acquiert pendant les 5 premières années de sa vie. C'est pendant cette période, et surtout entre 6 mois et 2 ans que le paludisme entraîne une mortalité dont l'estimation est difficile.

Aujourd'hui, prés de deux milliards d'êtres humains, souvent parmi les plus déshérités , vivent dans les zones d'endémies et sont exposés au risque paludéen; ils en subissent, souvent sans recours, les effets morbides ou même létaux. La protection de ces groupes à risques , plus du tiers de la population de la planète, constitue un défi pour la santé publique . Plusieurs centaines de millions de cas sont répertoriés chaque année. Plasmodium Falciparum est le parasite responsable de la plupart des cas de paludisme (80 % de cas dans le monde ) et des formes les plus graves , souvent mortelles, de la maladie. Les pays développés ne sont pas épargnés : le nombre de cas importés augmente en raison de la progression des transports internationaux.

La résistance de Plasmodium Falciparum aux médicaments constitue maintenant la menace la plus grave pour la lutte contre la maladie. Elle est apparue au début des années 60 en Thaïlande et en Colombie, puis s'est étendue et a atteint l'Afrique en 1978. Les déplacements de populations ont également joué un rôle dans l'apparition et l'extension de la résistant Plasmodium Falciparum chloroquino-résistant est aujourd'hui largement répandu dans le monde. La résistance à l'association de deuxième recours, Sulfamide/Pyrimethamine, est déjà répandue dans les secteurs chloroquino-réfractaires d'Asie du sud-est et d'Amérique du sud. L'émergence actuelle de polychimio-résistances à la totalité des antimalariques disponibles est redoutable.

Parmi les différentes méthodes de lutte contre le paludisme actuellement disponibles, on peut citer la lutte antilarvaire, et la réduction des sources qui restent à être analysées en terme de réduction de l'incidence des cas. Ces méthodes ne paraissent pas avoir un effet décisif sur le paludisme. Les traitements - intradomiciliaires par insecticides présentent de nombreuses insuffisances (résistance, réticences des populations aux traitements ou prix de revient augmentés, pas de résultats dans les savanes) tandis que les moustiquaires imprégnées par un pyréthrinoïde, réellement efficaces, présentent quelques limitations à l'usage.

Le vaccin polyvalent, pleinement actif contre les différentes formes du parasite et tous les types de paludisme, est sans cesse différé et apparaît comme lointain (Walsh J., Science 1987 , 235, 1319, Butcher, Parasitologie, 1989, 98, 315). La chimiothérapie demeurera, pour longtemps encore, une méthode de lutte nécessaire pour les populations des zones endémiques.

Un effort de recherche important entrepris à partir de 1963 à l'Institut américain Walter Reed ( Washington D.C. ), qui a testé plus de 250.000 molécules, a permis la mise sur le marché de Mefloquine (Lariam®) en 1985. Cependant, la résistance à ce nouvel antipaludéen a été induite expérimentalement. Des cas de résistance sur le terrain ont été rapportés et, faits plus graves, des résistances croisées entre méfloquine et autres amino-alcools comme la quinine ou de nouvelles drogues en expérimentation, tel qu'Halofantrine mise sur le marché en 1989 (Halfan®) ont été mises en évidence ( K. Rieckmann, Ann. Rev. Med, 1983, 34, 321-335 ; D. Warhust, Drugs, 1987 33, 50-65 ; Struchler, Parasitol, Today, 1989, 5, 39-40.).

Dans la classe des hématozoaires comparables à Plasmodium, on note Babésia, infestant particulièrement les animaux. Babésia est très ressemblant à Plasmodium mais infecte surtout les animaux comme les bovins ou le chien. Les parasites responsables de cette maladie sont principalement Babésia Bovis, Babésia cani, Babésia gibsoni, Babésia divergens, Babésia bigemina, Babésia equi est plus spécifiquement impliquée chez le cheval.

Les inventeurs ont mis en évidence une nouvelle classe de molécules possédant une activité antiprotozoaire élevée, en particulier antipaludéenne et antibabésiose. De plus cette activité antipaludéenne s'exerce par un mécanisme tout à fait original et pourrait ainsi éviter une pharmacorésistance toujours redoutée dans cette classe thérapeutique. La conception de ces molécules a été guidée par la mise en évidence d'un métabolisme phospholipidique abondant et spécifique dans le globule rouge impaludé. En effet, la prolifération asexuée intraérythrocytaire du parasite (phase associée aux symptômes cliniques de la maladie) s'accompagne d'une néosynthèse considérable de phospholipides (PL nécessaires à la biogenèse des membranes de Plasmodium. L'augmentation du taux de phospholipides intraérythrocytaire après impaludation atteint 500 % lorsque le parasite est au stade mature.

En conséquence, le métabolisme biosynthétique des phospholipides est pléthorique dans l'érythrocyte, après infection par Plasmodium. Il est complètement absent de l'érythrocyte hôte mature de mammifère qui est totalement dépourvu de la capacité de biosynthèse de PL.

Les différentes voies de biosynthèse des phosphatidylethanolamines (PE) et phosphatidylcholine (PC) sont schématisés dans la figure 1. La présente invention utilise l'observation du blocage du développement du parasite par

des substances interférant sur ce métabolisme à des doses très inférieures à celles interférant avec les cellules saines. Certains ammoniums quaternaires commerciaux et connus pour d'autres activités thérapeutiques ou non, inhibent le développement de Plasmodium Falciparum mais ceci à des doses les rendant inutilisables comme médicament en raison de leurs effets annexes notamment sur le système cholinergique. Ces sels quaternaires sont des trimethylalkylammoniums connus depuis longtemps comme tensioactifs, ou comme dépressants sur le muscle lisse (voir Decamethonium, Procuran®) et ceci par voie parentérale. ISOMAA (Acta Pharmacol; Toxicol 45 (5) 1979 et Biochem. Pharmacol 28 (7) 975/980) étudie l'action des alkyltrimethylammoniums quaternaires sur la membrane érythrocytaire du rat, et note un effet protecteur de la membrane à faible concentration et un effet hémolytique à dose plus élevée. Ces trimethylammoniums quaternaires à longue chaîne semblent induire une altération des surfaces cellulaires à concentration relativement élevée ( ISOMAA Acta Pharmacol. Toxicol. 44 (1) 1979 36/42 ). Le mécanisme d'action en modifiant la bicouche lipidique de la membrane plasmatique agirait plutôt selon un mécanisme tensioactif effectif à concentration relativement élevée.

Des séries bis trimethylalkylammoniums à chaîne linéaire longue ont été étudiées notamment pour leurs activités dépolarisantes sur divers modèles cellulaires (Kratskin L Gen. Pharmacol. 1980 11 (1) 119-26 et Skylarov Dokl. Akad. Nauk SSSR 1988 303 (3) 760-3). Les seules activités thérapeutiques notées sont de type cholinomimétique ou cholinolytique par Danilov A F Acta Physiol. Acta Sci. Hung. 1974 45 (3-4) 271-80). On note pour tous ces tensioactifs des groupes trimethylammoniums portés par chaque extrémité d'une chaîne hydrocarbonée généralement d'une longueur de douze enchaînements méthylène. L'atome d'azote supporte donc des substituants identiques peu encombrants sur une chaîne ne dépassant pas 12 atomes de carbone.

Les inventeurs ont trouvé de façon surprenante qu'en diminuant la symétrie autour de l'atome d'azote de composés bisamonium quaternaires et plus généralement en augmentant leurs encombrements stériques, on augmentait fortement l'inhibition du transporteur de choline chez le parasite. Cette dissymétrie autour de l'atome d'azote est notamment sensible pour les substituants les plus petits. Cette découverte surprenante conjuguée à l'ajustement de la longueur de la chaîne hydrocarbonée supportant ces deux têtes polaires des composés bisamoniums permet de multiplier l'activité sur Plasmodium par un facteur de 1000 voire 100.000 de dérivés connus à chaîne courte. La longueur optimum de la chaîne aliphatique espaçant les deux motifs ammoniums quaternaires influe sur cette activité antiplasmodium. L'altération de la transformation de la choline en phosphatidylcholine est spécifique du parasite hématozoaire notamment Plasmodium Falciparum aux concentrations actives utilisées. Il n'y a pas altération des autres systèmes biosynthétiques.

L'invention concerne donc des nouvelles molécules chimiques contenant deux groupes ammoniums quaternaires portés aux extrémités d'une chaîne hydrocarbonée, et exerçant une forte activité antipaludéenne et antibabésiose. Par activité antipaludéenne et antibabésiose, il faut entendre la capacité d'empêcher le développement du parasite à l'intérieur de l'érythrocyte et ou l'invasion érythrocytaire, et d'entraîner la mort des parasites initialement présents. Il est tout à fait remarquable que des composés de l'invention testés à la fois sur des souches chloroquine sensibles et sur des souches polypharmaco-résistantes présentent la même activité antipaludéenne.

Ainsi, l'invention concerne une composition pharmaceutique comprenant un excipient physiologiquement acceptable et, comme principe actif, un composé défini par la formule générale (I) suivante :

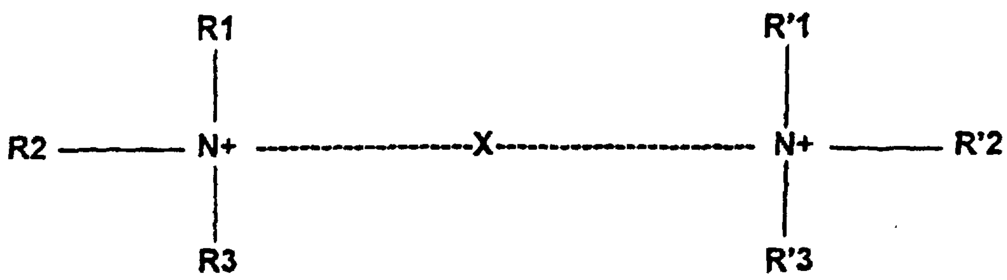

(I)

dans laquelle:

- R1 représente un groupe hydrocarboné ayant de 1 à 20 atomes de carbone, et
- R2 et R3 sont identiques ou différents et représentent indépendamment un groupe hydrocarboné ayant de 1 à 20

atomes de carbone éventuellement substitué soit par un groupe méthyle ou éthyle, soit par des groupes halogènes comme le chlore, le brome ou l'iode, soit par des groupes trifluorométhyl, triffuoroéthyl ou trifluoropropyl,

où R1 diffère du groupe éthyle, R2 et R3 n'étant pas simultanément méthyle,
ou

- R1 représente un groupement hydroxyalkyle comprenant de 1 à 5 atomes de carbone, les groupes hydroxyles pouvant être éventuellement éthérifés par substitution de l'atome d'hydrogène par un groupe methyl, ethyl, ou substitués par des dérivés silylés du type -Si-$(CH_3)_3$, et
- R2 et R3 sont identiques ou différents entre eux et représentent un groupe hydrocarboné saturé éventuellement substitué par des groupes halogènes comme le chlore, le brome, l'iode ou des groupes trifluoromethyl, trifluoroethyl, trifluoropropyl,

ou

- R1 représente un groupement hydrocarboné mono- ou polyinsaturé ayant de 1 à 6 atomes de carbone, et
- R2 et R3 sont identiques ou différents et représentent une chaîne alkyle linéaire ou ramifiée comportant de 1 à 20 enchaînements hydrocarbonés de, éventuellement substitués par un groupe methyl, ethyl ou par des halogènes comme le chlore, le brome, l'iode ou des motifs trifluoromethyl, trifluoethyl, trifluoropropyl,

ou

- R1 représente un groupement hydrocarboné ayant de 1 à 20 atomes de carbone, et
- R2 et R3 forment ensemble avec l'azote un hétérocycle azoté saturé comprenant 4 ou 5 atomes de carbone dans lequel un atome de carbone peut être remplacé par un atome de silicium, un atome d'oxygène ou un atome de soufre, ce cycle pouvant comporter en outre un substituant alkyle ayant de 1 à 3 atomes de carbone pouvant être lui-même hydroxyle, halogéné, ou silylé,

où R1', R2' et R3' ont la même définition que R1, R2 et R3 dans la formule (I), sans la restriction concernant les méthyles et les éthyles, les deux têtes polaires pouvant être identiques ou non ;
et où X représente une chaîne hydrocarbonée comportant éventuellement un substituant alkyle ayant 1 à 3 atomes de carbone, cet enchaînement hydrocarboné ayant de 12 à 26 atomes de carbone.

Préférentiellement, les deux têtes polaires des composés ci-dessus décrits sont identiques.

Plus particulièrement, les compositions pharmaceutiques selon l'invention comprennent un principe actif défini par la formule générale (I), dans laquelle, les substituants sont choisis selon les combinaisons suivantes :

- R1 représente un groupe alkyle choisi parmi les suivants:

  methyl, methylethyl, propyl, butyl, sec-butyl, tert-butyl, pentyl, hexyl, heptyl, octyl, nonyl, decyl, undecyl, dodecyl, tridécyl, tetradécyl, pentadécyl, hexadécyl, heptadécyl, octadécyl, nonadécyl, eicosyl, et

- R2 et R3 sont identiques ou différents et représentent indépendamment un groupe alkyle choisi parmi les suivants : ethyl, propyl, isopropyl, butyl, sec-butyl, tert-butyl, pentyl, hexyl, heptyl, octyl, nonyl, decyl, undecyl, dodecyl, tridécyl, tetradécyl, pentadécyl, hexadécyl, heptadécyl, octadécyl, nonadécyl, eicosyl éventuellement substitué soit par un groupe méthyle ou éthyle, soit par des groupes halogènes comme le chlore, le brome ou l'iode, soit par des groupes trifluorométhyl, trifluoroéthyl ou trifluoropropyl,

ou

- R1 est un groupement hydroxyalkyle comprenant de 1 à 5 atomes de carbone, notamment les groupes hydroxymethyl, hydroxyethyl, hydroxypropyl, hydroxybutyl, hydroxypentyl, ces groupes hydroxyles pouvant être éventuellement éthérifiés par substitution de l'atome d'hydrogène par un groupe methyl, ethyl, ou -Si-$(CH_3)_3$, et
- R2 et R3 sont identiques ou différents l'un de l'autre et peuvent représenter un groupement hydrocarboné saturé propyl, isopropyl, butyl, sec-butyl, tert-butyl,pentyl, hexyl, heptyl, octyl, nonyl, decyl, undecyl, dodecyl, éventuellement substitué par des halogènes comme le chlore, le brome, l'iode ou des groupes trifluoromethyl, trifluoroethyl, trifluoropropyl,

ou

- R1 représente une chaîne hydrocarbonée mono ou polyinsaturée du type propényl, propynyl, butényl, butynyl, butadiényl, pentènyl, pentadiényl, pentynyl, hexènyl, hexadiènyl, hexynyl, isoprènyl, et
- R2 et R3 sont identiques ou différents et représentent une chaîne alkyl linéaire ou ramifiée comportant de 1 à 20 enchaînements hydrocarbonés tel que methyl, ethyl, propyl, isopropyl, butyl, sec-butyl, tert-butyl,pentyl, hexyl, heptyl, octyl, nonyl, decyl, undecyl, dodecyl, tridécyl, tetradécyl, pentadécyl, hexadécyl, heptadécyl, octadécyl, nonadécyl, eicosyl, éventuellement substitués par un groupe methyl ou ethyl pouvant être lui-même substitué par des halogènes comme le chlore, le brome, l'iode ou des groupes trifluoromethyl, trifluoethyl, trifluoropropyl,

ou

- R1 représente un groupe methyl, methylethyl, propyl, butyl, sec-butyl, tert-butyl, pentyl, hexyl, heptyl, octyl, nonyl, decyl, undecyl, dodecyl, tridécyl, tetradécyl, pentadécyl, hexadécyl, heptadécyl, octadécyl, nonadécyl, eicosyl, et
- R2 et R3 forment ensemble avec l'azote un hétérocycle azoté saturé comprenant 4 ou 5 atomes de carbone dans lequel un atome de carbone peut être remplacé par un atome de silicium , un atome d'oxygène ou un atome de soufre, ce cycle pouvant comporter en outre un substituant du type methyl, ethyl, hydroxymethyl, hydroxyethyl, methyl ethyl, hydroxypropyl, un groupe trifluoromethyl, un groupe trifluoroethyl, ou un groupe trimethylsilyl, trimethylsilyloxy.

De manière préférée, X est ici une chaîne hydrocarbonée saturée de préférence linéaire, ayant entre 14 et 26, de préférence entre 14 et 22, par exemple 16 atomes de carbone.

Avantageusement, le principe actif est choisi parmi les composés suivants :

N, N'-dimethyl-N, N'-diethyl-N, N'-dipropyl-1,16-hexadecanediaminium, dibromure,
N,N'-dimethyl-N,N'-diethyl-N,N'-dipropyl-1,20-eicosanediaminium, dibromure,
N.N'-dimethyl-N,N,N',N'-tetraethyl-1,16-hexadecanediaminium dibromure,
N,N'-dimethyl-N, N, N', N'-tetraethyl-1.18-octadecanediaminium dibromure,
N,N'-dimethyl-N,N,N',N'-tetraethyl-1,21-heneicosanediaminium dibromure -1,1'-(1,14-tetradecanediyl) bis (1-methylpyrrolidinium ) dibromure, -1, 1'-( 1, 16-hexadecanediyl) bis (1 -methylpyrrolidinium) dibromure,
1,1'-(1,16-hexadecanediyl) bis (2-hydroxymethyl-1-methylpyrrolidinium dibromure,
N,N'-di-(2-hydroxyethyl) -N,N,N',N'-tetrapropyt-1,20-eicosanediaminium dibromure.

Dans le cas de l'hétérocycle azoté, R1 peut être un méthyle, pour fournir le type de tête polaire suivant :

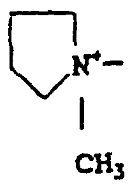

N-méthyl- pyrolidinium

Un composé constituant un exemple préféré de principe actif selon l'invention, et comportant deux de ces têtes polaires, est le - 1,1'- 1,16-hexadecanediyl bis (1-methylpyrrolidinium) dibromure.

L'invention concerne en outre un composé pour utilisation comme agent thérapeutique, de préférence en tant qu'agent anti-paludéen et/ou antibabésiose.

L'invention vise également l'utilisation d'un composé défini par la formule générale (I), pour l'obtention d'un médicament antipaludéen, ou antibabésiose :

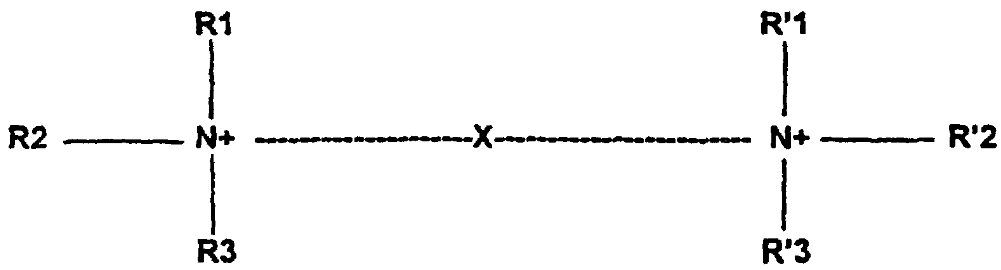

(I)

dans laquelle:

- R1 représente un groupe hydrocarboné ayant de 1 à 20 atomes de carbone, et
- R2 et R3 sont identiques ou différents et représentent indépendamment un groupe hydrocarboné ayant de 1 à 20 atomes de carbone éventuellement substitué soit par un groupe méthyle ou éthyle, soit par des groupes halogènes comme le chlore, le brome ou l'iode, soit par des groupes trifluorométhyl, trifluoroéthyl ou trifluoropropyl;

ou

- R1 représente un groupement hydroxyalkyle comprenant de 1 à 5 atomes de carbone, les groupes hydroxyles pouvant être éventuellement éthérifiés par substitution de l'atome d'hydrogène par un groupe methyl, ethyl, ou substitués par des dérivés silylés du type $-Si-(CH_3)_3$, et
- R2 et R3 sont identiques ou différents entre eux et représentent un groupe hydrocarboné saturé éventuellement substitué par des halogènes comme le chlore, le brome, l'iode ou des groupes trifluoromethyl, trifluoroethyl, trifluoropropyl ;

ou

- R1 représente un groupement hydrocarboné mono ou polyinsaturé ayant de 1 à 6 atomes de carbone, et
- R2 et R3 sont identiques ou différents et représentent une chaîne alkyl linéaire ou ramifiée comportant de 1 à 20 enchaînements hydrocarbonés, éventuellement substitués par un motif methyl, un ethyl ou par des halogènes comme le chlore, le brome, l'iode ou des motifs trifluoromethyl, trifluoethyl, trifluoropropyl ;

ou

- R1 représente un groupement hydrocarboné ayant de 1 à 20 atomes de carbone, et
- R2 et R3 forment ensemble avec l'azote un hétérocycle azoté saturé comprenant 4 ou 5 atomes de carbone dans lequel un atome de carbone peut être remplacé par un atome de silicium, un atome d'oxygène ou un atome de soufre, ce cycle pouvant comporter en outre un substituant alkyle ayant de 1 à 3 atomes de carbone pouvant être lui-même hydroxylé, halogéné, ou silylé;

où R1', R2' et R3' ont la même définition que R1, R2 et R3 dans la formule (I),
et où X représente une chaîne hydrocarbonée saturée contenant des groupements $-CH_2-$ ou des groupements cycliques comportant éventuellement un substituant alkyle ayant 1 à 3 atomes de carbone, cet enchaînement hydrocarboné ayant entre 11 et 26 atomes de carbone.

Selon cet aspect de l'invention, l'utilisation des composés correspondant aux principes actifs précédemment définis est plus particulièrement préférée.

L'invention concerne aussi un procédé pour la préparation d'une composition pharmaceutique caractérisé en ce qu'un principe actif tel que ci-dessus défini par la formule générale (I) est combiné avec un excipient physiologiquement acceptable.

En outre ce principe actif peut être combiné avec un additif conventionnel, tel qu'un agent conservateur, un agent antioxydant ou un diluant.

L'invention vise également les principes actifs de l'invention, tels que définis par la formule générale (I) relative aux compositions pharmaceutiques.

La présente invention concerne également un procédé de préparation des composés décrits. Les composés selon (I) peuvent être préparés selon les techniques connues . Parmi celles-ci, on peut citer l'action d'une amine tertiaire sur un alkane $\alpha$-$\omega$ dihalogéné soit un dichloro, un dibromo, ou un diiodo alkane . La réaction peut avoir lieu sans solvant de préférence sous azote, dans la mesure où l'un des réactifs au moins peut servir de milieu solvant. On peut aussi bien effectuer la réaction dans un solvant choisi parmi les alcools, les cétones, le Dimethylformamide, l'acétonitrile, les ethers, les ethers de polyglycol, ou le mélange de plusieurs solvants choisis parmi les précédents ou leur mélange avec des hydrocarbures aromatiques comme le toluène ou le benzène. On isole de cette façon l'halogénure d'ammonium quaternaire, par exemple le dibromure si l'on a utilisé un dibromoalkane.

La proportion des réactifs mis en présence est celle de la stoechiométrie, mais elle peut faire apparaître un excès de dérivé halogéné, notamment dans le cas où celui-ci peut être utilisé comme solvant réactionnel.

On peut cependant obtenir un sel d'un autre acide minéral ou organique par un procédé habituel, par échange sur une résine échangeuse d'ions . La réaction peut être effectuée à température ambiante, cependant on élèvera la température avantageusement pour accélérer son accomplissement. Cette température sera comprise entre l'ambiance et la température d'ébullition du milieu solvant utilisé.

Dans le cas où l'on veut obtenir un dérivé non symétrique, on fait réagir un équivalent d'amine tertiaire avec un $\omega$-alkanol primaire, puis par halogénation de la fonction alcool par l'acide halogénohydrique, par exemple l'acide bromhydrique concentré, on obtient un alkyl ammonium quaternaire $\omega$-halogéné, qui sera ensuite mis en réaction avec la seconde amine quaternaire choisie.

Le produit final peut être isolé directement par cristallisation à partir du milieu réactionnel, il pourra être également séparé du solvant par évaporation et recristallisé dans un autre solvant choisi parmi les alcools, les cétones, les esters, les ethers, les ether-alcools, l'acide acétique . Ces compositions peuvent être administrées par voie orale, par voie parentérale, rectale, percutanée ou permucosale, ceci sous la forme de comprimés, de dragées, capsules, solutions, sirops, émulsions, suspensions, ou de formes galéniques capables de moduler la libération du principe actif. De telles compositions peuvent être administrées à l'homme ou à l'animal à des doses comprises entre 0,01 mg/kg et 50 mg/kg.

Lors d'une application antibabésiose, les compositions pharmaceutiques sont formulées de manière appropriée pour une application vétérinaire.

Outre les dispositions qui précédent, l'invention concerne encore d'autres dispositions, qui ressortiront de la description qui va suivre , qui se réfère à des exemples de mise en oeuvre du procédé objet de la présente invention. Il est entendu toutefois que ces exemples sont donnés à titre d'illustration de l'objet de l'invention dont ils ne constituent en aucune manière une limitation. Tous les dérivés ont été soumis à l'analyse élémentaire, avec une tolérance maximale de 0,3 % dans les résultats obtenus, par rapport aux valeurs calculées. Les points de fusion ont été réalisés en tube capillaire et ne sont pas corrigés, les points d'ébullition n'ont pas été corrigés, la pression étant mesurée par une jauge de Pirani. Les spectres Infrarouge ont été réalisées sur un appareil Philipps PU 9714 en lames de NaCI pour les liquides et en pastilles de KBr pour les solides. Les spectres de RMN ont été enregistrés sur un appareil Brucker WP 200 en solution dans le Deutérochloroforme ou le DMSOd6 par rapport au TMS de référence. Sauf mention contraire, les produits ont été isolés à l'état de Bromhydrates.

## EXEMPLES

I/ SYNTHESE DE CERTAINES MOLECULES ACTIVES

EXEMPLE1:. -N, N'-dimethyl-N, N'-diethyl-N, N'-dipropyl-1, 14 tetradecanediaminium, dibromure.

On ajoute à 200 ml d'éthanol, 35,6 g ( 0.1 Mole ) de -1,14 dibromotetradecane et 11,1 g de N-ethyl-N-methyl-1-propanamine. On porte à reflux pendant 8 heures jusqu'à accomplissement de la réaction, suivi par Chromatographie en Couche Mince sur plaque de silice dans un système solvant comme Propanol/Pyridine/acide acétique/Eau. La solution est ensuite évaporée et le résidu est recristallisé dans un mélange Isopropanol/acétate d'éthyle. On obtient le dérivé du titre sous la forme de cristaux de pF = 185/190 °C .

Les dérivés $\alpha$-$\omega$ dibromés non commerciaux peuvent être synthétisés entre autres par une synthèse malonique à partir du dérivé dibromoalkane inférieur de quatre atomes de carbone. A titre d'exemple, on décrit ci-après la synthèse (a/, b/, c/ et d/) du -1,14-dibromotetradécane, à partir du dibromodécane :

a/ Synthèse de l'Ethyl ester de l'acide -2,13-diethoxycarbonyl-1,14-tétradécanedioique :

On dissout un atome/gramme de Sodium ( 23 g ) dans 500 ml d'éthanol anhydre, puis on introduit 1,1 mole

de Diethyl malonate. A la suspension résultante, on ajoute lentement 0.5 Mole de -1,10 dibromodécane entre 30 et 50 °C . On maintient le reflux quelques heures puis, après distillation et lavages à l'eau, on distille le produit brut . On obtient le dérivé du titre à l'état pur sous forme d'une huile distillant à 160 /170 °C sous $10^{-3}$ mBar avec un rendement de 80 %.

b/ Synthèse de l'acide -1,1 4-tetradecanedicarboxylique :

La totalité du produit obtenu a/ ci-dessus est saponifié dans un mélange de 170 g de Potasse et 200 ml d'eau. L'alcool produit est distillé puis le milieu est fortement acidifié par de l'acide sulfurique concentré. On porte à l'ébullition jusqu'à la fin de décarboxylation. Quand tout le $CO_2$ est dégagé, on refroidit et extrait par un solvant non miscible qu'on lave jusqu'à neutralité. Après évaporation et recristallisation, on obtient le dérivé du titre de pF = 160 °C

c/ Synthèse du -1,14-Tetradecanediol :

Le produit de l'essai précédent b/ est estérifié en présence d'un excès d'éthanol et de quelques millilitres d'acide sulfurique porté à l'ébullition. L'avancement de la réaction peut être suivi en C.C.M. dans le système solvant suivant : Acide Formique / Formiate d'éthyle / Toluène. Après évaporation de l'excès d'alcool et lavage à l'eau en présence d'un solvant non miscible, on obtient l'ester éthylique de l'acide -1,14-tetradecanedioique sous forme d'une huile épaisse de Péb = 165 °C sous 1 mbar. La totalité du produit est réduite par $LiAlH_4$ en solution dans le THF, l'avancement de la réduction est suivi par CCM . Après traitement par l'acide dilué, le produit est extrait par un solvant puis chromatographie sur colonne de Silice par le mélange hexane/acétate d'éthyle. Le rendement total à partir du diacide est supérieur à 65 %.

d/ Synthèse du -1,14-dibromotetradecane :

On introduit 23 g ( 0.1 mole) de dialcool dans 200 ml d'acide bromhydrique à 48 % et porte à reflux 24 heures. Après refroidissement, on sépare la couche organique, on la redissout dans le chloroforme et on lave à l'eau. Par chromatographie sur colonne de Silice avec de l'hexane, on obtient le dérivé du titre avec un rendement de 45 % par rapport à l'alcool ; le point de fusion du produit est de 43 °C.

En pratiquant comme précédemment décrit dans l'exemple 1, et avec les dibromoalkanes correspondants à la place du -1,14-dibromotetradecane, on obtient les dérivés suivants :

EXEMPLE 2 : N,N'-dimethyl-N, N'-diethyl-N, N'-dipropyl-1, 15-pentadecanediaminium, dibromure de pF = 230 °C.

EXEMPLE 3 : N.N'-dimethyl-N.N'-diethyl-N, N'-dipropyl-1,16-exadecanediaminium, dibromure de pF = 232 °C.

EXEMPLE 4 : N,N'-dimethyl-N, N'-diethyl-N, N'-dipropyl-1, 17-heptadecanediaminium, dibromure de pF = 205 °C .

EXEMPLE 5 : N,N'-dimethyl-N,N'-diethyl-N,N'-dipropyl-1,18-octadecanediaminium, dibromure de pF = 175 °C.

EXEMPLE 6 : N, N'-dimethyl-N, N'-diethyl-N, N'-dipropyl-1,20-eicosanediaminium, dibromure de pF = 130 °C.

EXEMPLE 7 : N,N'-dimethyl-N, N'-diethyl-N, N'-dipropyl-1,22-Cosanediaminium, dibromure de pF = 130 °C .

EXEMPLE 8 : -N, N, N, N', N', N' hexapropyl-1, 14-tetradecanediaminium, dibromure.

On ajoute 3,56 g ( 10 mM ) de -1,14-dibromotetradecane dans 20 ml de tripropylamine et porte à 80/90 °C pendant une nuit. On évapore sous vide l'excès d'amine tertiaire, on reprend par le minimum d'éthanol et on reprécipite à l'éther. Après recristallisation dans le mélange Isopropanol/éther isopropylique; on obtient le dérivé du titre de pF =170 °C.

EXEMPLE 9 : -N, N, N, N', N', N' hexapropyl-1, 16-hexadecanediaminium, dibromure.

On opère comme ci-dessus avec le -1,16-dibromohexadecane en recristallisant dans Méthanol/ éther, on obtient le dérivé du titre sous forme hygroscopique de pF= 185 °C .

EXEMPLE 10: -N, N, N, N', N', N' hexapropyl-1,20-eicosanediaminium, dibromure.

La synthèse malonique décrite à l'exemple 1 est appliquée au -1,16-dibromohexadecane pour aboutir au -1,20-eicosandiol obtenu à l'état pur après chromatographie sur colonne avec un pF = 93 °C . La bromation selon l'exemple 1 - d - variante 2 aboutit au -1,20-dibromoeicosane de pF = 62 °C . La réaction avec la tripropylamine selon l'exemple 1 aboutit au dérivé du titre sous forme cristallisée ayant un pF = 205 °C.

EXEMPLE 11 : -N, N, N, N', N', N' hexapropyl-1,24-tetracosanediaminium, dibromure.

La synthèse malonique est appliquée cette fois au dérivé bromé décrit ci-dessus et aboutit au -1,24-dibromotetracosane, qui traité par tripropylamine selon l'exemple 1 donne le dérivé du titre de pF = 105 °C.

EXEMPLE 12 : - N, N, N, N', N', N' hexapropyl-1,16-hexadecanediaminium, dibromure.

A partir du dérivé -1,16-dibromohexadécane et la tripropylamine dans les conditions ci-dessus, on obtient le dérivé du titre sous forme cristallisée de pF = 227 °C.

EXEMPLE 13 : N,N'-dimethyl-N,N,N',N'-tetraethyl-1,16-hexadecanediaminium, dibromure.

En substituant la N-methyldiethylamine à la tripropylamine, on obtient le dérivé du titre de pF = 170 °C.

EXEMPLE 14 : N,N'-dimethyl-N,N,N',N'-tetraethyl-1,18 octadecanediaminium, dibromure.

On opère comme ci-dessus avec du dérivé -1,18dibromooctadecane et l'on obtient le produit cité de pF = 206'C.

EXEMPLE 15 : N,N'-dimethyl-N,N,N',N'-tetraethyl-1,21-heneicosanediaminium, dibromure.

A partir du diol correspondant obtenu selon Lukes ( Col. Czech. Chem. Comm. 26 , -1961- 1719/1722 ) et action du tribromure de phosphore selon l'exemple 1, on obtient le dérivé α-ω dibromé que l'on fait réagir comme ci-dessus sur la N-methyldiethylamine pour aboutir au dérivé du titre de pF = 205 °C.

EXEMPLE 16 : -1,1'-( 1,14-tetradecanediyl) bis ( 1-methylpyrrolidinium) dibromure.

On ajoute 21.36 g ( 0.06 mole ) de -1,14-dibromodecane et 11.07 g (0.13 mole ) de N-methylpyrrolidine à 200 millilitres d'éthanol, porte à reflux 6 heures jusqu'à fin de réaction. Après évaporation à sec et recristallisation dans un mélange éthanol/éther , on obtient le dérivé du titre de pF = 192 °C.

EXEMPLE 17 : -1,1-( 1,16-hexadecanediyl) bis (1-methylpyrrolidinium) dibromure.

Dans les conditions de l'exemple ci-dessus et avec le -1,16 dibromodécane, on obtient avec la N-methylpyrrolidine, le dérivé du titre sous forme cristalline de PF = 178 °C.

EXEMPLE 18: 1,1'-( 1, 16-hexadecanediyl) bis (2-hydroxymethyl-1-methylpyrrolidinium) dibromure.

Si dans l'exemple précédent, on substitue la N-methylpyrrolidine par de la 2-hydroxymethyl-1-methyl-pyrrolidine, on obtient le dérivé du titre de pF = 95 °C.

EXEMPLE 19 : 1,1'-(1,22 -docosanediyl) bis (2-hydroxymethyl1-methylpyrrolidinium) dibromure.

A partir du 1,22-dibromodocosane, préparé à partir du dialcool correspondant, lui-même synthétisé par action de l'acide bromhydrique sur le docosa-1,21-diène, on obtient le dérivé du titre de pF = 102 °C.

EXEMPLE 20 : -1,1'-(1,16-hexadecanediyl) bis (1-methyl-3hydroxy methyl piperidinium) dibromure.

Dans les conditions de l'exemple 16 avec du -1,16-dibromohexadecane et du 1-methyl-3-piperidinemethanol, on obtient le dérivé cité de pF = 107 °C.

EXEMPLE 21 : 1,1'-( 1.16-hexadecanediyl) bis (1-methylmorpholinium) dibromure.

En utilisant le même dérivé bromé et la N-methylmorpholine, on obtient le dérivé du titre de p = 85 °C.

EXEMPLE 22 : N,N'-didodecyl-N,N,N',N'-tetramethyl- 1,16 hexadecanediaminium dibromure.

L'utilisation de la N-Dimethyllaurylamine avec le dérivé dibromé ci-dessus aboutit au dérivé du titre sous forme d'un produit blanc de pF = 128 °C.

EXEMPLE 23 : N, N'-didodecyl-N, N, N', N'-tetramethyl-1,16 octadecanediaminium dibromure.

Comme ci-dessus avec le dérivé dibromo en C18, on obtient le dérivé recherché de PF = 103 °C.

EXEMPLE 24 : -N,N'-di (2-propynyl)-N,N,N',N'-tetramethyl-1,14-tetradecane diaminium dibromure.

On utilise la N-dimethylpropargylamine dans les conditions précédentes et l'on obtient le dérivé du titre de pF=145°C le spectre de RMN enregistré dans le DMSOd6 présente les déplacements caractéristiques suivants : 3,4 ppm (m), 4H, -N-CH2-CH2-. 4,1 ppm; 4,1 ppm (t), 2H, CH ≡C-; 4,5 ppm (d) 4H, C-CH2-N-.

EXEMPLE 25 : -N,N'-di (2-propenyl )-N,N,N',N'-tetramethyl-1,14 tetradecane diaminium dibromure.

Comme ci-dessus avec la N-dimethylallylamine, on obtient le produit ci-dessus de pF = 50 / 60 °C. Les déplacements chimiques caractéristiques en RMN sont- 4,0 ppm (d), 4H, CH-CH2-N- 5,6 ppm (m) 4H, CH2=CH-; 6,0 ppm (m) 2H, CH2=CH-CH2.

EXEMPLE 26: -N,N'-di (3-butynyl)-N, N, N', N'-tetramethyl 1,14-tetradecanediaminium dibromure.

De la même façon que ci-dessus avec la maineacétylénique correspondante, on obtient le dérivé du titre de pF = 170 °C. Son spectre RMN présente les déplacements chimiques suivants : 2,75 ppm, (td), 4H, CH≡C-CH2-CH2-N , 3,1 ppm (t) 2H, CH ≡-C-CH2 ; 3,25 ppm (m) 4H, CH≡C-CH2-CH2-N-.

EXEMPLE 27: -N, N'-di-(2-hydroxyethyl) -N, N, N', N'-tetrapropyl-1, 14 tetradecane diaminium dibromure.

A partir de N-dipropylethanolamine et du dérivé bromé en C14, on obtient le dérivé du titre de pF = 188 / 190 °C.

EXEMPLE 28 : -N, N'-di-(2-ethoxyethyl)-N, N, N', N'-tetrapropyl-1, 14 tetradecanediaminium dibromure.

En opérant comme ci-dessus à partir de N-dipropyl-2-ethoxyethylamine, on obtient le dérivé du titre, de pF = 145/150 °C.

EXEMPLE 29 : -N, N'-di-(2-hydroxyethyl) -N, N, N', N'-tetrapropyl-1, 1 6 hexadecane diaminium dibromure.

On opère selon l'exemple 27 avec du -1,16-dibromohexadecane au lieu de -1,14-tetradecane et on obtient le dérivé recherché de pF = 120 °C.

EXEMPLE 30: N, N'-di-(2-hydroxyethyl)-N, N, N', N'-tetrapropyl-1,20-eicosanediaminium dibromure.

Par action du dérivé dibromé en C20 selon l'exemple précédent on obtient le dérivé cité de pF = 85/90 °C.

II/ ACTIVITE ANTIPALUDEENNE

L'activité antipaludéenne des dérivés selon les exemples précédents est testée sur l'humain infesté par Plasmodium falciparum. Le produit à tester est mis en contact avec les érythrocytes humains infectés pendant 24 heures. Un précurseur radioactif d'acides nucléiques l'Hypoxanthine ($^3$H) est ensuite ajouté. Le précurseur ne s'incorpore que dans les cellules infectées par un parasite en croissance, c'est à dire dans les cellules que la drogue n'aura pas affectées.

L'aptitude ou non à incorporer le précurseur reflète donc la viabilité des cellules impaludées. La durée des tests est de 60 à 70 heures ( test de DESJARDINS R.E. , CANFIELD C.J., HATNES J.D. et CHULAY J.D., Antimicrob.

Agents Chemother. 1979, 16, p 710-718 ). Les résultats sont consignés dans le tableau I ci-après, sous forme de IC50 (ou DE50), soit la concentration de produit dans le milieu expérimental capable d'inhiber in vitro la croissance du parasite d'une valeur de 50%.

La vérification du mécanisme d'action est réalisée sur les divers dérivés selon l'invention par l'étude de l'interférence spécifique avec la biosynthèse des différentes biomolécules, acides nucléiques, protéines et phospholipides à partir de l'incorporation de précurseurs radiomarqués soit ($^3$H) Hypoxanthine, ($^3$H) Isoleucine, ($^3$H) Choline. La spécificité au sein même du métabolisme phospholipidique est déterminée par comparaison avec l'effet sur l'incorporation de ($^3$H) Ethanolamine dans la phosphatidyléthanolamine (Ancelin M.L. Vialettes F. et Vial H.J. 1991, Anal. Biochem. 199, 203-209).

Activité antipaludique de G25 chez le singe infecté par le parasite humain P. falciparum :

En dehors des chimpanzés, seules deux espèces de singes d'Amérique du Sud, Aotus et Saimiri Scirius, ont pu être infecté par P. falciparum. L'infection des singes Aotus par le parasite humain offre actuellement le meilleur et quasi-unique modèle pour l'évaluation des approches thérapeutiques ou vaccinales du paludisme (Collins W.E, Gallaud C.G. Sullivan J.S., and Morris C.L, « Selections of different strains of Plasmodium falciparum for testing blood stages in Aotus Nancymai monkeys ». An. J. Trop. Med. Hyg., 1994, 51 (2) 224-232).

L'activité antipaludique a donc été évaluée chez le singe Aotus Lemurinus infecté par l'isolat FVO (falciparum Vietnam Oaknoll) de P. falciparum. Cet isolat est chloroquine résistant et est invariablement létal pour les singes Aotus de la «Fundacion Centro de Primates » de l'Université del Valle, Cali, Colombia.

Ainsi que le montre la Figure 6, le singe Aotus est infecté par P. falciparum (isolat FVO) au jour 0. Lorsque la parasitémie atteint 5,6%, le traitement par G25 (dissous dans NaCl 9%) a été initié à raison de 16 doses de 0,2 mg/kg (2 administrations par jour pendant 8 jours). La décroissance de la parasitémie est apparente dès la deuxième dose. Pour comparaison, la parasitémie d'un singe traité par le mélange sulfadoxine/pyriméthamine (FANSIDAR) est représentée.

Au total , 15 singes ont été infectés puis traités par G25 donné en intramusculaire à des doses allant de 0,010 à 0,2 mg/kg. Des guérisons complètes (vérifiées par PCR) et sans recrudescence (observation pendant 6 mois et contrôle par la susceptibilité à une nouvelle infection), ont été observés à des doses aussi faibles que 0,030 mg/kg. A 0,01 mg/kg, G25 est actif (clearance de la parasitémie) mais ne conduit pas à une guérison complète (observation de recrudescence).

Considérant la dose maximum tolérable chez le singe (voisine de 1,5 mg/kg, il s'ensuit que l'index thérapeutique ($DL_{50}/DE_{50}$) chez le singe est supérieur à 50.

III / ACTIVITE ANTIPALUDEENNE ET TOXICITE

Afin d'évaluer l'index thérapeutique des divers produits, on a mesuré l'activité de ceux-ci dans un test *in vivo* et comparé à la toxicité aiguë chez l'animal . Cette activité est mesurée suivant le test décrit par PETERS W. ( Chemotherapy and Drug résistance in Malaria, 1970). Un composé est administré pendant quatre jours consécutifs à des souris préalablement impaludées par Plasmodium vinckei, petteri, ou chabaudi, ledit composé étant dissous dans une solution de NaCl à 0.9 %. Les préparations sont ainsi administrées par voie intrapéritonéale ou par voie sous-cutanée à des souris Swiss mâles infectées par voie IV, avec Plasmodium petterei ou Plasmodium chabaudi ($10^6$ cellules infectées ). Le composé est administré deux fois par jour pendant 4 jours consécutifs, la première injection étant pratiquée 2 heures après l'infestation et la deuxième, 10 heures après. La parasitémie est déterminée par un frottis, le jour suivant la fin du traitement. La toxicité est mesurée *in vivo* selon la même méthode, les animaux ayant reçu dans les conditions d'administration ci-dessus, 2 injections par jour pendant 4 jours ( toxicité semi chronique). Les résultats sont exprimés sous forme de DL 50, soit la dose ayant entraîné la mort de 50% des animaux.

Les résultats des essais sont résumés dans le tableau I ci-après sous forme d'index thérapeutique, soit le rapport de l'activité à la toxicité mésurées dans des conditions identiques comme décrit ci-dessus.

IV/ SPECIFICITE D'ACTION

1- Les molécules selon l'invention ont une action spécifique sur le métabolisme des phospholipides par rapport au métabolisme des acides nucléiques et, au sein du métabolisme phospholipidique. Les tests d'activité in vitro sont pratiqués sur les érythrocytes humains infestés par Plasmodium Falciparum. Les précurseurs radioactifs utilisés tels que la choline, l'ethanolamine ou l'hypoxanthine, sont ajoutés et permettent de mesurer l'effet du produit sur ces divers métabolismes. Tous les composés testés présentent une spécificité d'action sur la biosynthèse de

la phosphatidylcholine, avec une étroite corrélation entre l'action sur ce métabolisme et l'action antipaludéenne proprement dite.

2- Les molécules selon l'invention ont montré à l'inverse une absence totale d'action par rapport à d'autres systèmes cellulaires. Ainsi leurs effets sur la viabilité de la lignée lymphoblastoide SAR ont été mesurés : il n'existe aucune corrélation entre la concentration conduisant à une inhibition de 50 % de la viabilité de la lignée cellulaire LV50 et la IC50 relative à Plasmodium Falciparum. Cette même IC50 a été comparée à l'IC 50 nécessaire à inhiber l'entrée de la choline dans les synaptosomes du système nerveux central selon Tamaru (Brain Res. 473, 205-226). L'effet sur le système nerveux ne se manifeste qu'à des doses 100 à 1000 fois supérieures.

V / ACTIVITE ANTIBABESIA IN VITRO

Le procédé de criblage d'activité anti-malaria, basé sur l'incorporation de l'($^3H$) Hypoxanthine par le parasite, est utilisé pour tester l'activité anti-métabolique de tels composés sur Babesia Bovis, Babesia Canis. Une relation étroite est trouvée entre le taux d'incorporation de ($^3H$) Hypoxanthine dans une mesure standard et le pourcentage de cellules parasitées, déterminé par examen microscopique. Cette activité métabolique est quantifiée et évaluée dans le tableau ci-dessous par la cotation +++ pour les molécules affichant dans le test une inhibition de 50 % de l'incorporation de ($^3H$) Hypoxanthine (ID 50), ces molécules étant à une concentration inférieure ou égale à 0,01 Micromolaire. La cotation est de ++ pour la même activité à une concentration de 0,01 à 0.1 micromolaire et enfin de + pour une concentration inférieure ou égale à 1 Micromolaire.

Tableau I :

| Composés | IC50 exprimée en micromoles | Index thérapeutique | Activité/ Babesia à 10 millimole |
|---|---|---|---|
| EXEMPLE1 | 0,01 | 50 | ++ |
| EXEMPLE 2 | 0,01 | 42 | + |
| EXEMPLE 3 | 0,003 | 15 | ++ |
| EXEMPLE 4 | 0,002 | 17 | ++ |
| EXEMPLE 5 | 0,001 | 12 | +++ |
| EXEMPLE 6 | 0,003 | 15 | ++ |
| EXEMPLE 7 | 0,01 | 35 | ++ |
| EXEMPLE 8 | 0,1 | 20 | + |
| EXEMPLE 9 | 0,007 | 36 | +++ |
| EXEMPLE 10 | 0,7 | 14 | + |
| EXEMPLE 11 | 1,6 | 28 | + |
| EXEMPLE 12 | 0,001 | 11 | ++ |
| EXEMPLE 13 | 0,0005 | 6 | +++ |
| EXEMPLE 14 | 0,00004 | 14 | +++ |
| EXEMPLE 15 | 0,000003 | 12 | +++ |
| EXEMPLE 16 | 0,0009 | 8 | +++ |
| EXEMPLE 17 | 0,0006 | 7 | +++ |
| EXEMPLE 18 | 0,001 | 22 | +++ |
| EXEMPLE 19 | 0,07 | 40 | + |
| EXEMPLE 20 | 0,0003 | 17 | +++ |
| EXEMPLE 21 | 0,001 | 25 | +++ |
| EXEMPLE 22 | 1 | 20 | ++ |
| EXEMPLE 23 | 0,1 | 35 | + |
| EXEMPLE 24 | 0,007 | 12 | ++ |

Tableau I : (suite)

| Composés | IC50 exprimée en micromoles | Index thérapeutique | Activité/ Babesia à 10 millimole |
|---|---|---|---|
| EXEMPLE 25 | 0,005 | 15 | +++ |
| EXEMPLE 26 | 0,04 | 45 | ++ |
| EXEMPLE 27 | 0,01 | 15 | ++ |
| EXEMPLE 28 | 0,01 | 40 | +++ |
| EXEMPLE 29 | 0,005 | 12 | ++ |
| EXEMPLE 30 | 0,0003 | 55 | +++ |

LEGENDE DES FIGURES:

Figure 1 : Schéma des différentes voies de biosynthèse de phosphatidylethanolamine (PE) et de phosphatidylcholine (PC).

Figure 2 : Corrélation entre l'activité antipaludéenne (IC50) et l'action sur le métabolisme phospholipidique (PL50) (r = 0,86, ce qui correspond à un risque très inférieur à 0.01 %). LV50 représente une inhibition de 50% de la viabilité cellulaire.

Figure 3 : Voies de synthèse de bisammoniums quaternaires (dépend de «n»).

Figure 4 : Réactions faisant intervenir des synthèses maloniques.

Figure 5 : Synthèse de dérivés bisammoniums comprenant des groupements acétyléniques et ethyléniques.

Figure 6 : Effet antipaludique de G25 chez le singe infecté par P. falciparum à parasitémie de 6%.

**Revendications**

**1.** Composition pharmaceutique comprenant un excipient physiologiquement acceptable et, comme principe actif, un composé défini par la formule générale (I) suivante:

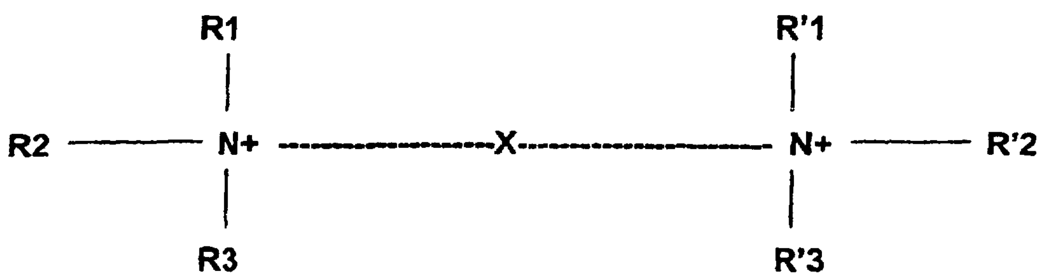

(I)

dans laquelle:

- R1 représente un groupe hydrocarboné ayant de 1 à 20 atomes de carbone, et

- R2 et R3 sont identiques ou différents et représentent indépendamment un groupe hydrocarboné ayant de 1 à 20 atomes de carbone éventuellement substitué soit par un groupe méthyle ou éthyle, soit par des groupes halogènes comme le chlore, le brome ou l'iode, soit par des groupes trifluorométhyl, trifluoroéthyl ou trifluoropropyl ;

où R1 diffère du groupe ethyl, R2 et R3 n'étant pas simultanément méthyle,
ou

- R1 représente un groupement hydroxyalkyle comprenant de 1 à 5 atomes de carbone, les groupes hydroxyles pouvant être éventuellement éthérifiés par substitution de l'atome d'hydrogène par un groupe methyl, ethyl, ou substitués par des dérivés silylés du type $-Si-(CH_3)_3$, et
- R2 et R3 sont identiques ou différents entre eux et représentent un groupe hydrocarboné saturé éventuellement substitué par des groupes halogènes comme le chlore, le brome, l'iode ou des groupes trifluoromethyl, trifluoroethyl, trifluoropropyl ;

ou

- R1 représente un groupement hydrocarboné mono ou polyinsaturé ayant de 1 à 6 atomes de carbone, et
- R2 et R3 sont identiques ou différents et représentent une chaîne alkyle linéaire ou ramifiée comportant de 1 à 20 enchaînements hydrocarbonés de, éventuellement substitués par un groupe methyl, ethyl ou par des halogènes comme le chlore, le brome, l'iode ou des motifs trifluoromethyl, trifluoethyl, trifluoropropyl;

ou

- R1 représente un groupement hydrocarboné ayant de 1 à 20 atomes de carbone, et
- R2 et R3 forment ensemble avec l'azote un hétérocycle azoté saturé comprenant 4 ou 5 atomes de carbone dans lequel un atome de carbone peut être remplacé par un atome de silicium, un atome d'oxygène ou un atome de soufre, ce cycle pouvant comporter en outre un substituant alkyle ayant de 1 à 3 atomes de carbone pouvant être lui-même hydroxyle, halogéné, ou silylé;

où R1', R2' et R3' ont la même définition que R1, R2 et R3 dans la formule (1), sans la restriction concernant les méthyles et les éthyles, les deux têtes polaires pouvant être identiques ou non;
et où X représente une chaîne hydrocarbonée comportant éventuellement un substituant alkyle ayant 1 à 3 atomes de carbone, cet enchaînement hydrocarboné ayant de 12 à 26 atomes de carbone, excepté les composés suivants :

$(CH_3)_3\text{-}N^+\text{-}(CH_2)_{16}\text{-}N^+\text{-}(CH_3)_3\ 2Br^-$

$(HOCH_2CH_2)(CH_3)_2\text{-}N^+\text{-}(CH_2)_{18}\text{-}N^+\text{-}(CH_3)_2(CH_2CH_2OH)\ 2Br^-$

$(ROCH_2CH_2)(CH_3CH_2)_2\text{-}N\text{-}(CH_2)_{18}\text{-}N\text{-}(CH_2CH_3)_2(CH_2CH_2OH)\ 2Br$

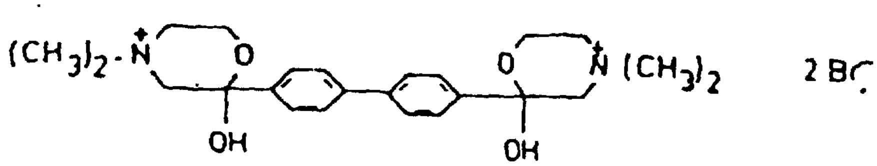

2. Composition pharmaceutique selon la revendication 1 dans laquelle:

- R1 représente un groupe alkyle choisi parmi les suivants :
methyl, methylethyl, propyl. butyl, sec-butyl, tert-butyl, pentyl, hexyl, heptyl, octyl, nonyl, decyl, undecyl, dodecyl, tridécyl, tetradécyl, pentadécyl, hexadécyl, heptadécyl, octadécyl, nonadécyl, eicosyl, et
- R2 et R3 sont identiques ou différents et représentent indépendamment un groupe alkyle choisi parmi les suivants : ethyl, propyl, isopropyl, butyl, sec-butyl, tert-butyl, pentyl, hexyl, heptyl, octyl, nonyl, decyl, undecyl, dodecyl, tridécyl, tetradécyl, pentadécyl, hexadécyl, heptadécyl, octadécyl, nonadécyl, eicosyl éventuellement substitué soit par un groupe méthyle ou éthyle, soit par des groupes halogènes comme le chlore, le brome ou l'iode, soit par des groupes trifluorométhyl, trifluoroéthyl ou trifluoropropyl.

**3.** Composition pharmaceutique selon la revendication 1 dans laquelle:

- R1 est un groupement hydroxyalkyle comprenant de 1 à 5 atomes de carbone, notamment les groupes hydroxymethyl, hydroxyethyl, hydroxypropyl, hydroxybutyl, hydroxypentyl, ces groupes hydroxyles pouvant être éventuellement éthérifiés par substitution de l'atome d'hydrogène par un groupe methyl, ethyl, ou -Si-$(CH_3)_3$, et
- R2 et R3 sont identiques ou différents l'un de l'autre et peuvent représenter un groupement hydrocarboné saturé propyl, isopropyl, butyl, sec-butyl, tert-butyl,pentyl, hexyl, heptyl, octyl, nonyl, decyl, undecyl, dodecyl, éventuellement substitué par des halogènes comme le chlore, le brome, l'iode ou des groupes trifluoromethyl, trifluoroethyl, trifluoropropyl.

**4.** Composition pharmaceutique selon la revendication 1 dans laquelle

- R1 représente une chaîne hydrocarbonée mono ou polyinsaturée du type propényl, propynyl, butényl, butynyl, butadiényl, pentènyl, pentadiényl, pentynyl, hexènyl, hexadiényl, hexynyl, isoprènyl, et - R2 et R3 sont identiques ou différents et représentent une chaîne alkyl linéaire ou ramifiée de 1 à 20 enchaînements hydrocarbonés tel que methyl, ethyl, propyl, isopropyl, butyl, sec-butyl, tert-butyl,pentyl, hexyl, heptyl, octyl, nonyl, decyl, undecyl, dodecyl, tridécyl, tetradécyl, pentadécyl, hexadécyl, heptadécyl, octadécyl, nonadécyl, eicosyl, éventuellement substitués par un groupe methyl ou ethyl pouvant être lui-même substitué par des halogènes comme le chlore, le brome, l'iode ou des groupes trifluoromethyl, trifluoethyl, trifluoropropyl.

**5.** Composition pharmaceutique selon la revendication 1 dans laquelle

- R1 représente un groupe methyl, methylethyl, propyl, butyl, sec-butyl, tert-butyl, pentyl, hexyl, heptyl, octyl, nonyl, decyl, undecyl, dodecyl, tridécyl, tetradécyl, pentadécyl, hexadécyl, heptadécyl, octadécyl, nonadécyl, eicosyl, et
- R2 et R3 forment ensemble avec l'azote un hétérocycle azoté saturé comprenant 4 ou 5 atomes de carbone dans lequel un atome de carbone peut être remplacé par un atome de silicium , un atome d'oxygène ou un atome de soufre, ce cycle pouvant comporter en outre un substituant du type methyl, ethyl, hydroxymethyl, hydroxyethyl, methyl ethyl, hydroxypropyl, un groupe trifluoromethyl, un groupe trifluoroethyl, ou un groupe trimethylsilyl, trimethylsilyloxy.

**6.** Composition pharmaceutique selon l'une quelconque des revendications 1 à 5 dans laquelle X représente une chaîne hydrocarbonée saturée de préférence linéaire, et ayant de préférence entre 14 et 26, de préférence 14 et 22, par exemple 16 atomes de carbone.

**7.** Composition pharmaceutique selon l'une quelconque des revendications 1 à 6 dans laquelle les deux têtes polaires sont identiques.

**8.** Composition pharmaceutique selon l'une quelconque des revendications 1 à 6 **caractérisée en ce que** le principe actif est choisi parmi les composés suivants :

N,N'-dimethyl-N,N'-diethyl-N,N'-dipropyl-1,16-hexadecanediaminium, dibromure ;
N,N'-dimethyl-N,N'-diethyl-N,N'-dipropyl-1,20-eicosanediaminium, dibromure ;
N,N'-dimethyl-N,N,N',N'-tetraethyl-1,16-hexadecanediaminium dibromure ;
N,N'-dimethyl-N,N,N',N'-tetraethyl-1,18-octadecanediaminium dibromure ;
N,N'-dimethyl-N,N,N',N'-tetraethyl-1,21-heneicosanediaminium dibromure ;
- 1,1'-(1,1 4-tetradecanediyl) bis (1 -methylpyrrolidinium ) dibromure ;
-1,1'-( 1,16-hexadecanediyl) bis (1-methylpyrrolidinium) dibromure ;
-1,1'-(1,16-hexadecanediyl) bis (2-hydroxymethyl-1-methylpyrrolidinium dibromure ;
N,N'-di-(2-hydroxyethyl) -N,N,N',N'-tetrapropyl-1,20-eicosanediaminium dibromure.

**9.** Utilisation d'un composé défini par la formule générale (I), pour l'obtention d'un médicament antipaludéen, ou antibabésiose :

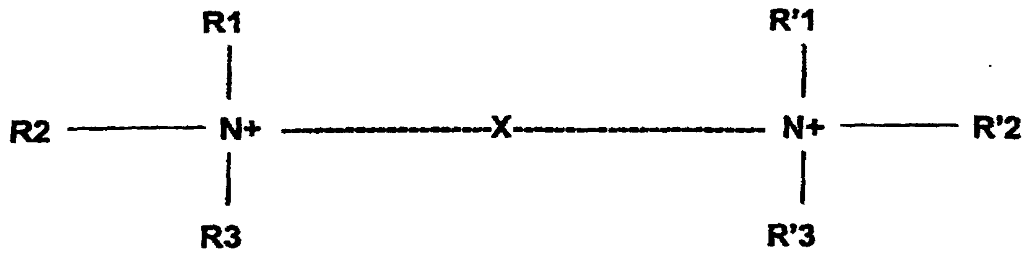

(I)

dans laquelle:

- R1 représente un groupe hydrocarboné ayant de 1 à 20 atomes de carbone, et
- R2 et R3 sont identiques ou différents et représentent indépendamment un groupe hydrocarboné ayant de 1 à 20 atomes de carbone éventuellement substitué soit par un groupe méthyle ou éthyle, soit par des groupes halogènes comme le chlore, le brome ou l'iode, soit par des groupes trifluorométhyl, trifluoroéthyl ou trifluoropropyl ;

ou

- R1 représente un groupement hydroxyalkyle comprenant de 1 à 5 atomes de carbone, les groupes hydroxyles pouvant être éventuellement éthérifiés par substitution de l'atome d'hydrogène par un groupe methyl, ethyl, ou substitués par des dérivés silylés du type $-Si-(CH_3)_3$, et
- R2 et R3 sont identiques ou différents entre eux et représentent un groupe hydrocarboné saturé éventuellement substitué par des halogènes comme le chlore, le brome, l'iode ou des groupes trifluoromethyl, trifluoroethyl, trifluoropropyl ;

ou

- R1 représente un groupement hydrocarboné mono- ou polyinsaturé ayant de 1 à 6 atomes de carbone, et
- R2 et R3 sont identiques ou différents et représentent une chaîne alkyl linéaire ou ramifiée comportant de 1 à 20 enchaînements hydrocarbonés, éventuellement substitués par un motif methyl, un ethyl ou par des halogènes comme le chlore, le brome, l'iode ou des motifs trifluoromethyl, trifluoethyl, trifluoropropyl;

ou

- R1 représente un groupement hydrocarboné ayant de 1 à 20 atomes de carbone, et
- R2 et R3 forment ensemble avec l'azote un hétérocycle azoté saturé comprenant 4 ou 5 atomes de carbone dans lequel un atome de carbone peut être remplacé par un atome de silicium, un atome d'oxygène ou un atome de soufre, ce cycle pouvant comporter en outre un substituant alkyle ayant de 1 à 3 atomes de carbone pouvant être lui-même hydroxylé, halogéné, ou silyle;

où R1', R2' et R3' ont la même définition que R1, R2 et R3 dans la formule (I),
et où X représente une chaîne hydrocarbonée saturée contenant des groupements $-CH_2-$ ou des groupements cycliques comportant éventuellement un substituant alkyle ayant 1 à 3 atomes de carbone, cet enchaînement hydrocarboné ayant entre 11 et 26 atomes de carbone.

**10.** Utilisation d'un composé selon la revendication 9 **caractérisé en ce qu'**il correspond au principe actif définis dans les revendications 1 à 8.

**11.** Composé ayant la formule :

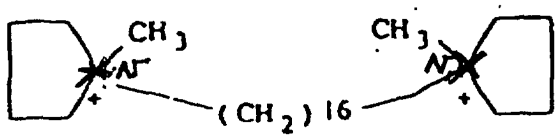

**12.** Procédé pour la préparation d'une composition pharmaceutique **caractérisé en ce qu'**un principe actif défini par la formule générale (1) selon l'une quelconque des revendications 1 à 8 et 10 est combiné avec un excipient physiologiquement acceptable.

**13.** Procédé selon la revendication 12 **caractérisé en ce que** le principe actif est en outre combiné avec un additif conventionnel, tel qu'un agent conservateur, un agent antioxydant ou un diluant.

**Patentansprüche**

**1.** Pharmazeutische Zubereitung die einen physiologisch annehmbaren Trägerstoff (Excipienz) und als wirksames Prinzip eine Verbindung der folgenden allgemeinen Formel (Gemisch 1) enthält:

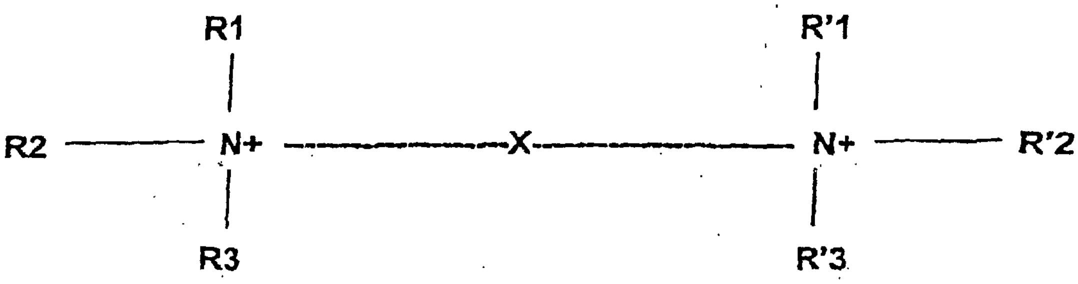

(I)

worin:

- R1 eine Kohlenwasserstoffgruppe mit 1 bis 20 Kohlenstoffatomen bedeutet und

- R2 und R3 identisch oder verschieden sind und unabhängig voneinander eine Kohlenwasserstoffgruppe mit 1 bis 20 Kohlenstoffatomen bedeuten, die gegebenenfalls entweder durch eine Methyl- oder Ethylgruppe, oder durch Halogengruppen, wie Chlor, Brom oder Iod, oder durch Trifluoromethyl-, Trifluoroethyl- oder Trifluoropropylgruppen substituiert ist;

worin R1 verschieden von der Ethyl-Gruppe ist, R2 und R3 nicht gleichzeitig Methyl bezeichnend, oder

- R1 eine Hydroxyalkyl-Gruppe mit 1 bis 5 Kohlenstoffatomen bedeutet, worin die Hydroxyl-Gruppen gegebenenfalls durch Substitution des Wasserstoffatoms durch eine Methyl-, Ethyl-Gruppe verethert, oder durch Silyl-Derivate von Typ-Si-$(CH_3)_3$ substituiert sein können und

- R2 und R3 identisch oder voneinander verschieden sind und eine gesättigte Kohlenwasserstoffgruppe bedeuten, die gegebenenfalls durch Halogen-Gruppen, wie Chlor, Brom, Iod, oder Trifluoromethyl-, Trifluoroethyl-, Trifluoropropyl-Gruppen substituiert ist;

oder

- R1 eine einfach oder mehrfach ungesättigte Kohlenwasserstoff-Gruppe mit 1 bis 6 Kohlenstoffatomen bedeutet und

- R2 und R3 identisch oder verschieden sind und eine lineare oder verzweigte A1-kylkette mit 1 bis 20 Kohlenwasserstoffverknüpfüngen bedeuten, gegebenenfalls substituiert durch eine Methyl-, Ethyl-Gruppe oder durch Halogene, wie Chlor, Brom, Iod, oder Trifluoromethyl-, Trifluoroethyl-, Trifluoropropyl-Reste; oder

- R1 eine Kohlenwasserstoffgruppe mit 1 bis 20 Kohlenstoffatomen bedeutet und

- R2 und R3 miteinander mit dem Stickstoff einen gesättigten Stickstoff-Heterozyklus mit 4 oder 5 Kohlenstoffatomen bilden, worin ein Kohlenstoffatom durch ein Siliciumatom, ein Sauerstoffatom oder ein Schwefelatom ersetzt sein kann und dieser Ring außerdem einen Alkyl-Substituenten mit 1 bis 3 Kohlenstoffatomen tragen kann, der selbst hydroxiliert, halogeniert oder silyliert sein kann.;

worin R1', R2' und R3' die gleiche Bedeutung wie R1, R2 und R3 in der Formel (I) haben ohne die Methyl-Gruppen und die Ethyl-Gruppen betreffende Einschränkung, wobei die zwei polaren Köpfe identisch oder nicht identisch sein können; und
worin X eine Kohlenwasserstoffkette bedeutet, die gegebenenfalls einen Alkyl-Substituienten mit 1 bis 3 Kohlenstoffatomen aufweist, wobei diese Kohlenstoffkette 12 bis 26 Kohlenstoffatome hat, ausgenommen die folgenden Verbindungen

$(CH_3)_3\text{-}\overset{+}{N}\text{-}(CH_2)_{16}\text{-}\overset{+}{N}\text{-}(CH_3)_3\ 2Br^-$

$(HOCH_2CH_2)(CH_3)_2\text{-}\overset{+}{N}\text{-}(CH_2)_{18}\text{-}\overset{+}{N}\text{-}(CH_3)_2(CH_2CH_2OH)\ 2Br^-$

$(RO\ CH_2CH_2)(CH_3CH_2)_2\text{-}\overset{+}{N}\text{-}(CH_2)_{18}\text{-}\overset{+}{N}\text{-}(CH_2CH_3)_2(CH_2CH_2OH)\ 2Br^-$

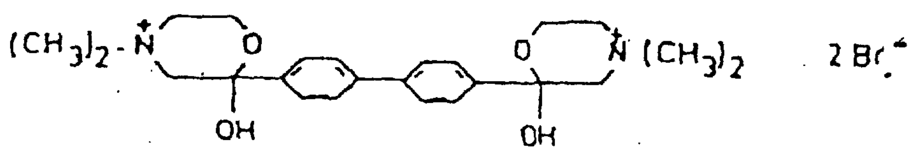

**2.** Pharmazeutische Zubereitung nach Anspruch 1, worin

- R1 eine Alkylgruppe bedeutet, die ausgewählt ist unter den folgenden: Methyl, Methylethyl, Propyl, Butyl, sec-Butyl, tert-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl und

- R2 und R3 identisch oder verschieden sind und unabhängig voneinander eine Alkylgruppe bedeuten, die ausgewählt ist aus den folgenden: Ethyl, Propyl, Isopropyl, Butyl, sec-Butyl, tert-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, gegebenenfalls substituiert durch entweder eine Methyloder Ethyl-Gruppe oder durch Halogen-Gruppen, wie Chlor, Brom oder Iod, oder durch Trifluoromethyl-, Trifluoroethyl- oder Trifluoropropyl-Gruppen.

**3.** Pharmazeutische Zubereitung nach Anspruch 1 worin

- R1 eine Hydroxyalkyl-Gruppe mit 1 bis 5 Kohlenstoffatomen ist, insbesondere die Gruppen Hydroxymethyl, Hydroxyethyl, Hydroxypropyl, Hydroxybutyl, Hydroxypentyl, wobei diese Hydroxyl-Gruppen gegebenenfalls durch Substitution des Wasserstoffatoms durch eine Methyl-, Ethyl- oder Si-$(CH_3)_3$-Gruppe verethert sein können und

- R2 und R3 identisch oder voneinander verschieden sind und eine gesättigte Kohlenwasserstoff-Gruppe, Propyl, Isopropyl, Butyl, sec-Butyl, tert-Butyl,Pentyl, Hexyl, Heptyl, Octyl, Nonyl, Decyl, Undecyl, Dodecyl bedeuten, die gegebenenfalls durch Halogene, wie Chlor, Brom, Iod, oder Trifluoromethyl-, Trifluoroethyl-, Trifluoropropyl-Gruppen substituiert ist.

**4.** Pharmazeutische Zubereitung nach Anspruch 1 worin

- R1 eine einfach oder mehrfach ungesättigte Kohlenwasserstoffkette vom Typ Propenyl, Propinyl, Butenyl, Butinyl, Butadienyl, Pentenyl, Pentadienyl, Pentinyl, Hexenyl, Hexadienyl, Hexinyl, Isoprenyl, bedeutet und

- R2 und R3 identisch oder verschieden sind und eine lineare oder verzweigte Alkylkette mit 1 bis 20 Kohlenwasserstoffverknüpfungen wie Methyl, Ethyl, Propyl, Isopropyl, Butyl, sec-Butyl, tert-Butyl,Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl bedeuten, die gegebenenfalls durch eine Methyl- oder Ethyl-Gruppe substituiert sein können, die selbst durch Halogene, wie Chlor, Brom, Iod, oder Trifluoromethyl-, Trifluoroethyl-, Trifluoropropyl-Gruppen substituiert sein können.

**5.** Pharmazeutische Zubereitung nach Anspruch 1, worin

- R1 eine Methyl, Methylethyl-, Propyl-, Butyl-, sec-Butyl-, tert-Butyl-, Pentyl-, Hexyl-, Heptyl-, Octyl-, Nonyl-, Decyl-, Undecyl-, Dodecyl-, Tridecyl-, Tetradecyl-, Pentadecyl-, Hexadecyl-, Heptadecyl-, Octadecyl-, Nonadecyl-, Eicosyl-Gruppen bedeutet und

- R2 und R3 zusammen mit dem Stickstoff einen gesättigten stickstoffhaltigen Heterozyklus mit 4 oder 5 Kohlenstoffatomen bilden, worin ein Kohlenstoffatom ersetzt sein kann durch ein Siliciumatom, ein Sauerstoffatom oder ein Schwefelatom und dieser Zyklus außerdem einen Substituenten vom Typ Methyl, Ethyl, Hydroxymethyl, Hydroxyethyl, Methylethyl, Hydroxypropyl, eine Triofluoromethyl-Gruppe, eine Trifluoroethyl-Gruppe oder eine Trimethylsilyl-, Trimethylsilyloxy-Gruppe aufweisen kann.

**6.** Pharmazeutische Zubereitung nach einem der Ansprüche 1 bis 5, worin X eine gesättigte vorzugsweise lineare Kohlenwasserstoffkette bedeutet und vorzugsweise zwischen 14 und 26, bevorzugt 14 und 22, beispielsweise 16 Kohlenstoffatome hat.

**7.** Pharmazeutische Zubereitung nach einem der Ansprüche 1 bis 6, worin die zwei polaren Köpfe identisch sind.

**8.** Pharmazeutische Zubereitung nach einem der Ansprüche 1 bis 6, **dadurch gekennzeichnet, daß** das aktive Prinzip ausgewählt ist aus den folgenden Verbindungen:

N,N'-Dimethyl- N,N'-Diethyl- N,N'-Dipropyl-1,16-Hexadecandiaminium-dibromid;
N,N'-Dimethyl- N,N'-Diethyl- N,N'-Dipropyl-1,20-Eicosandiaminium-dibromid;
N,N'-Dimethyl-N,N,N',N'-Tetraethyl-1,16-Hexadecandiaminium-dibromid;
N,N'-Dimethyl-N,N,N',N'-tetraethyl-1,18-octadecandiaminium-dibromid;
N,N'-Dimethyl-N,N,N',N'-tetraethyl-1,21-heneicosandiaminium-dibromid;
-1,1'-(1,1 4-Tetradecandiyl) bis ( 1-methylpyrrolidinium )-dibromid;
-1,1'-(1, 16-Hexadecandiyl) bis (1-methylpyrrolidinium)-dibromid;
-1,1'-(1,16-Hexadecandiyl) bis (2-hydroxymethyl-1-methylpyrrolidinium)-dibromid;
N,N'-Di-(2-hydroxyethyl) -N,N,N',N',-tetrapropyl-1,20-eicosandiaminium)dibromid;

**9.** Verwendung einer Verbindung mit der allgemeinen Formel (I) zur Herstellung eines Antimalaria- oder Antibabesiosis-Medikaments

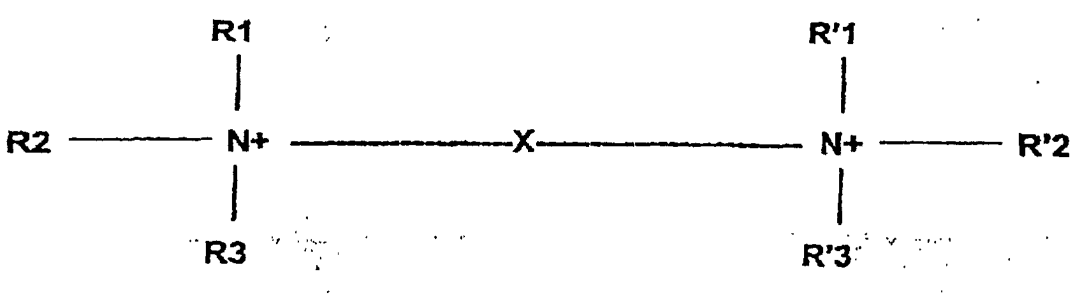

(I)

worin

- R1 eine Kohlenwasserstoff-Gruppe mit 1 bis 20 Kohlenstoffatomen bedeutet und

- R2 und R3 gleich oder verschieden sind und unabhängig voneinander eine Kohlenwasserstoff-Gruppe mit 1 bis 20 Kohlenstoffatomen bedeuten, die gegebenenfalls substituiert ist durch entweder eine Methyl- oder Ethyl-Gruppe oder durch Halogen-Gruppen, wie Chlor, Brom oder Iod oder durch Trifluoromethyl-, Trifluoroethyl- oder Trifluoropropyl-Gruppen;
oder

- R1 eine Hydroxyalkyl-Gruppe mit 1 bis 5 Kohlenstoffatomen bedeutet, wobei die Hydroxyl-Gruppen gegebenenfalls durch Substitution des Wasserstoffatoms durch eine Methyl-, Ethyl-Gruppe verethert oder durch Silylderivate vom Typ Si-$(CH_3)_3$ substituiert sein können und

- R2 und R3 gleich oder untereinander verschieden sind und eine gesättigte Kohlenwasserstoff-Gruppe bedeuten, die gegebenenfalls durch Halogene, wie Chlor, Brom, Iod, oder Trifluoromethyl-, Trifluoroethyl-, Trifluoropropyl-Gruppen substituiert ist;oder

- R1 eine einfach oder mehrfach ungesättigte Kohlenwasserstoff-Gruppe mit 1 bis 6 Kohlenstoffatomen bedeutet und

- R2 und R3 gleich oder verschieden sind und eine lineare oder verzweigte Alkylkette mit 1 bis 20 Kohlenwasserstoffverknüpfüngen bedeuten, die gegebenenfalls durch eine Methyl-Gruppe, eine Ethyl-Gruppe oder durch Halogene, wie Chlor, Brom, Iod, oder Trifluoromethyl-, Trifluoroethyl- oder Trifluoropropyl-Gruppen substituiert sind; oder

- R1 eine Kohlenwasserstoff-Gruppe mit 1 bis 20 Kohlenstoffatomen bedeutet und

- R2 und R3 miteinander mit dem Stickstoff einen stickstoffhaltigen gesättigten Heterozyklus mit 4 oder 5 Kohlenstoffatomen bilden, worin ein Kohlenstoffatom durch ein Siliciumatom, ein Sauerstoffatom oder ein Schwefelatom ersetzt sein kann und dieser Zyklus außerdem einen Alkylsubstituenten mit 1 bis 3 Kohlenstoffatomen aufweisen kann, der selbst hydroxyliert, halogeniert oder silyliert ist;

worin
R1', R2' und R3' die gleiche Bedeutung wie R1, R2 und R3 in der Formel (I) haben und
worin X eine gesättigte Kohlenwasserstoffkette bedeutet, welche Gruppen $-CH_2-$ oder zyklische Gruppen mit gegebenenfalls einem Alkylsubsituenten mit 1 bis 3 Kohlenstoffatomen aufweist, wobei diese Kohlenwasserstoffverknüpfung zwischen 11 und 26 Kohlenstoffatomen hat.

10. Verwendung einer Verbindung nach Anspruch 9, **dadurch gekennzeichnet, daß** sie dem in den Ansprüchen 1 bis 8 definiertem aktiven Prinzip entspricht.

11. Verbindung mit der Formel

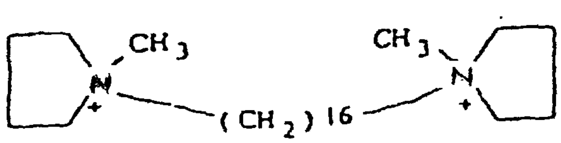

12. Verfahren zur Herstellung einer pharmazeutischen Zubereitung, **dadurch gekennzeichnet, daß** ein aktives Prinzip, das durch die allgemeine Formel (I) gemäß einem der Ansprüche 1 bis 8 und 10 definiert ist, mit einem physiologisch annehmbaren Trägerstoff(Exipienz) kombiniert wird.

13. Verfahren nach Anspruch 12, **dadurch gekennzeichnet, daß** das aktive Prinzip außerdem mit einem üblichen Zusatzstoff, wie einem Konservierungsmittel, einem Antioxidanz oder einem Verdünungsmittel kombiniert wird.

## Claims

1. Pharmaceutical composition containing a physiologically compatible excipient and, as active principle, a compound

defined by the following general formula (I):

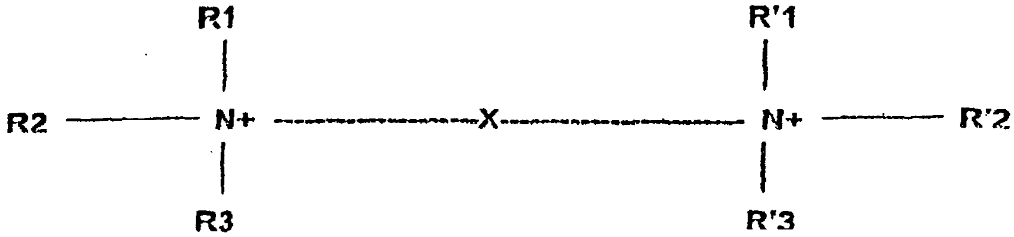

(I)

in which:

- R1 represents a hydrocarbon group having 1 to 20 carbon atoms and
- R2 and R3 are identical or different and represent independently of one another a hydrocarbon group having 1 to 20 carbon atoms optionally substituted by a methyl or ethyl group, or by halogen groups such as chlorine, bromine or iodine, or by trifluoromethyl, trifluoroethyl or trifluoropropyl groups,

and R1 differs from the ethyl group, R2 and R3 not simultaneously denoting methyl;
or

- R1 represents an hydroxyalkyl group containing 1 to 5 carbon atoms, the hydroxyl groups optionally being able to be etherified by replacement of the hydrogen atom by a methyl or ethyl group or substituted by silyl derivatives of the type $-Si-(CH_3)_3$, and
- R2 and R3 are identical or different and represent a saturated hydrocarbon group optionally substituted by halogen groups such as chlorine, bromine or iodine, or by trifluoromethyl, trifluoroethyl or trifluoropropyl groups;

or

- R1 represents a monounsaturated or polyunsaturated hydrocarbon group having 1 to 6 carbon atoms, and
- R2 and R3 are identical or different and represent a linear or branched alkyl chain containing 1 to 20 hydrocarbon linkages, optionally substituted by a methyl or ethyl group or by halogen groups such as chlorine, bromine or iodine, or by trifluoromethyl, trifluoroethyl or trifluoropropyl groups;

or

- R1 represents a hydrocarbon group having 1 to 20 carbon atoms, and
- R2 and R3 form together with nitrogen a saturated nitrogen-containing heterocycle containing 4 or 5 carbon atoms in which a carbon atom may be replaced by a silicon, oxygen or sulphur atom, this heterocycle moreover being able to contain an alkyl substituent having 1 to 3 carbon atoms that in turn may be able to be hydroxylated, halogenated or silylated,

where R1', R2' and R3' have the same meanings as R1, R2 and R3 in formula (1) without the restriction concerning the methyl and ethyl groups, the two polar terminal groups being able to be identical or not,
and where X represents a hydrocarbon chain optionally containing an alkyl substituent having 1 to 3 carbon atoms, this hydrocarbon linkage having 12 to 26 carbon atoms, with the exception of the following compounds:
$(CH_3)_3\text{-}N^+\text{-}(CH_2)_{16}\text{-}N^+\text{-}(CH_3)_3\ 2Br^-$
$(HOCH_2CH_2)(CH_3)_2\text{-}N^+\text{-}(CH_2)_{18}\text{-}N^+\text{-}(CH_3)_2(CH_2CH_2OH)\ 2Br^-$
$(RO\ CH_2CH_2)(CH_3CH_2)_2\text{-}N\text{-}(CH_2)_{18}\text{-}N\text{-}(CH_2CH_3)_2(CH_2CH_2OH)\ 2Br^-$

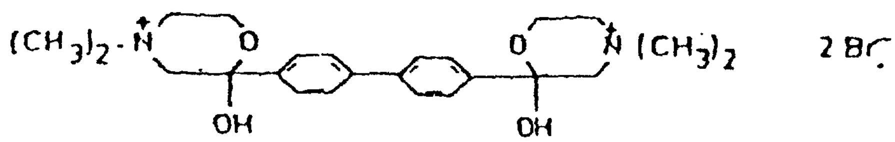

**2.** Pharmaceutical composition according to claim 1 in which:

- R1 represents an alkyl group selected from the following:

  methyl, methylethyl, propyl, butyl, sec.-butyl, tert.-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, and

- R2 and R3 are identical or different and represent independently of one another an alkyl group selected from the following: ethyl, propyl, isopropyl, butyl, sec.-butyl, tert.-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, optionally substituted either by a methyl or ethyl group, or by halogen groups such as chlorine, bromine or iodine, or by trifluoromethyl, trifluoroethyl or trifluoropropyl groups.

**3.** Pharmaceutical composition according to claim 1 in which:

- R1 is an hydroxyalkyl group containing 1 to 5 carbon atoms, in particular the hydroxymethyl, hydroxyethyl, hydroxypropyl, hydroxybutyl or hydroxypentyl groups, these hydroxyl groups optionally being able to be etherified by replacement of the hydrogen atom by a methyl, ethyl or $-Si-(CH_3)_3$ group, and
- R2 and R3 are identical of different from one another and may represent a saturated hydrocarbon group selected from propyl, isopropyl, butyl, sec.-butyl, tert.-butyl, pentyl, hexyl, heptyl, octyl, nonyl, decyl, undecyl, dodecyl, optionally substituted by halogen groups such as chlorine, bromine or iodine, or by trifluoromethyl, trifluoroethyl or trifluoropropyl groups.

**4.** Pharmaceutical composition according to claim 1, in which:

- R1 represents a monounsaturated or polyunsaturated hydrocarbon chain of the type selected from propenyl, propynyl, butenyl, butynyl, butadienyl, pentenyl, pentadienyl, pentynyl, hexenyl, hexadienyl, hexynyl, isoprenyl, and R2 and R3 are identical or different and represent a linear or branched alkyl chain containing 1 to 20 hydrocarbon linkages such as methyl, ethyl, propyl, isopropyl, butyl, sec.-butyl, tert.-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, optionally substituted by a methyl or ethyl group that may itself be substituted by halogen groups such as chlorine, bromine or iodine, or by trifluoromethyl, trifluoroethyl or trifluoropropyl groups.

**5.** Pharmaceutical composition according to claim 1, in which:

- R1 represents a methyl, methylethyl, propyl, butyl, sec.-butyl, tert.-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl group, and
- R2 and R3 form together with nitrogen a saturated nitrogen-containing heterocycle containing 4 or 5 carbon atoms in which a carbon atom may be replaced by a silicon, oxygen or sulphur atom, this heterocycle moreover being able to be substituted by methyl, ethyl, hydroxymethyl, hydroxyethyl, methylethyl, hydroxypropyl, by a trifluoromethyl or trifluoroethyl group, or by a trimethylsilyl or trimethylsilyloxy group.

**6.** Pharmaceutical composition according to any one of claims 1 to 5 in which X represents a saturated, preferably linear hydrocarbon chain preferably containing between 14 and 26, more preferably between 14 and 22 carbon atoms, for example 16 carbon atoms.

**7.** Pharmaceutical composition according to any one of claims 1 to 6 in which the two polar terminal groups are identical.

**8.** Pharmaceutical composition according to any one of claims 1 to 6, **characterised in that** the active principle is selected from the following compounds:

N,N' -dimethyl-N,N'-diethyl-N,N'-dipropyl-1, 16-hexadecanediaminium dibromide;
N,N'-dimethyl-N,N'-diethyl-N,N'-dipropyl-1,20-eicosanediaminium dibromide;
N,N'-dimethyl-N,N,N',N'-tetraethyl-1,16-hexadecane-diaminium dibromide;
N,N'-dimethyl-N,N,N',N'-tetraethyl-1,18-octadecane-diaminium dibromide;
N,N'-dimethyl-N,N,N',N'-tetraethyl-1,21-heneicosane-diaminium dibromide;
1,1'-(1,14-tetradecanediyl) bis(1-methylpyrrolidinium) dibromide;
1,1'-(1,16-hexadecanediyl) bis(1-methylpyrrolidinium) dibromide;
1,1'-(1,16-hexadecanediyl) bis(2-hydroxymethyl-1-methylpyrrolidinium) dibromide;
N,N'-di-(2-hydroxyethyl)-N,N,N',N'-tetrapropyl-1,20-eicosanediaminium dibromide.

**9.** Use of a compound defined by the general formula (I) for obtaining an anti-malarial or anti-babesiasis drug:

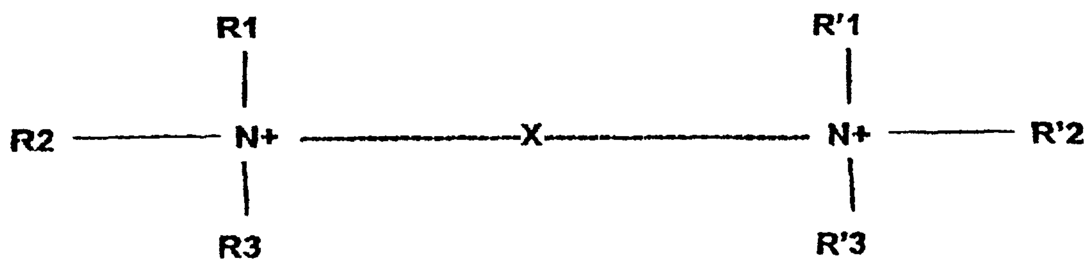

(I)

in which:

- R1 represents a hydrocarbon group having 1 to 20 carbon atoms, and
- R2 and R3 are identical or different and independently represent a hydrocarbon group having 1 to 20 carbon atoms optionally substituted either by a methyl or ethyl group, or by halogen groups such as chlorine, bromine or iodine, or by trifluoromethyl, trifluoroethyl or trifluoropropyl groups;

or

- R1 represents an hydroxyalkyl group containing 1 to 5 carbon atoms, the hydroxyl groups optionally being able to be etherified by replacement of the hydrogen atom by a methyl or ethyl group or substituted by silyl derivatives of the type $-Si-(CH_3)_3$, and
- R2 and R3 are identical or different and represent a saturated hydrocarbon group optionally substituted by halogen groups such as chlorine, bromine or iodine, or by trifluoromethyl, trifluoroethyl or trifluoropropyl groups;

or

- R1 represents a monounsaturated or polyunsaturated hydrocarbon group having 1 to 6 carbon atoms, and
- R2 and R3 are identical or different and represent a linear or branched alkyl chain containing 1 to 20 hydrocarbon linkages, optionally substituted by a methyl or ethyl group or by halogen groups such as chlorine, bromine or iodine, or by trifluoromethyl, trifluoroethyl or trifluoropropyl groups;

or
R1 represents an hydrocarbon group having 1 to 20 carbon atoms, and

- R2 and R3 form together with nitrogen a saturated nitrogen-containing heterocycle containing 4 or 5 carbon atoms in which a carbon atom may be replaced by a silicon, oxygen or sulphur atom, wherein this heterocycle may moreover contain an alkyl substituent having 1 to 3 carbon atoms that may in turn be hydroxylated, halogenated or silylated;

where

R1', R2' and R3' have the same meanings as R1, R2 and R3 in formula (I), and where X represents a saturated hydrocarbon chain containing $-CH_2-$ groups or cyclic groups optionally containing an alkyl substituent having 1 to 3 carbon atoms, this hydrocarbon linkage having between 11 and 26 carbon atoms.

**10.** Use of a compound according to claim 9, **characterised in that** it corresponds to the active principle defined in claims 1 to 8.

**11.** Compound having the formula

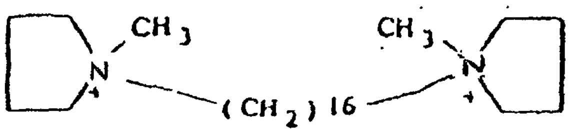

**12.** Process for the preparation of a pharmaceutical composition, **characterised in that** an active principle defined by the general formula (1) according to any one of claims 1 to 8 and 10 is combined with a physiologically compatible excipient.

**13.** Process according to claim 12, **characterised in that** the active principle is moreover combined with a conventional additive such as a preservative, an antioxidant or a diluent.

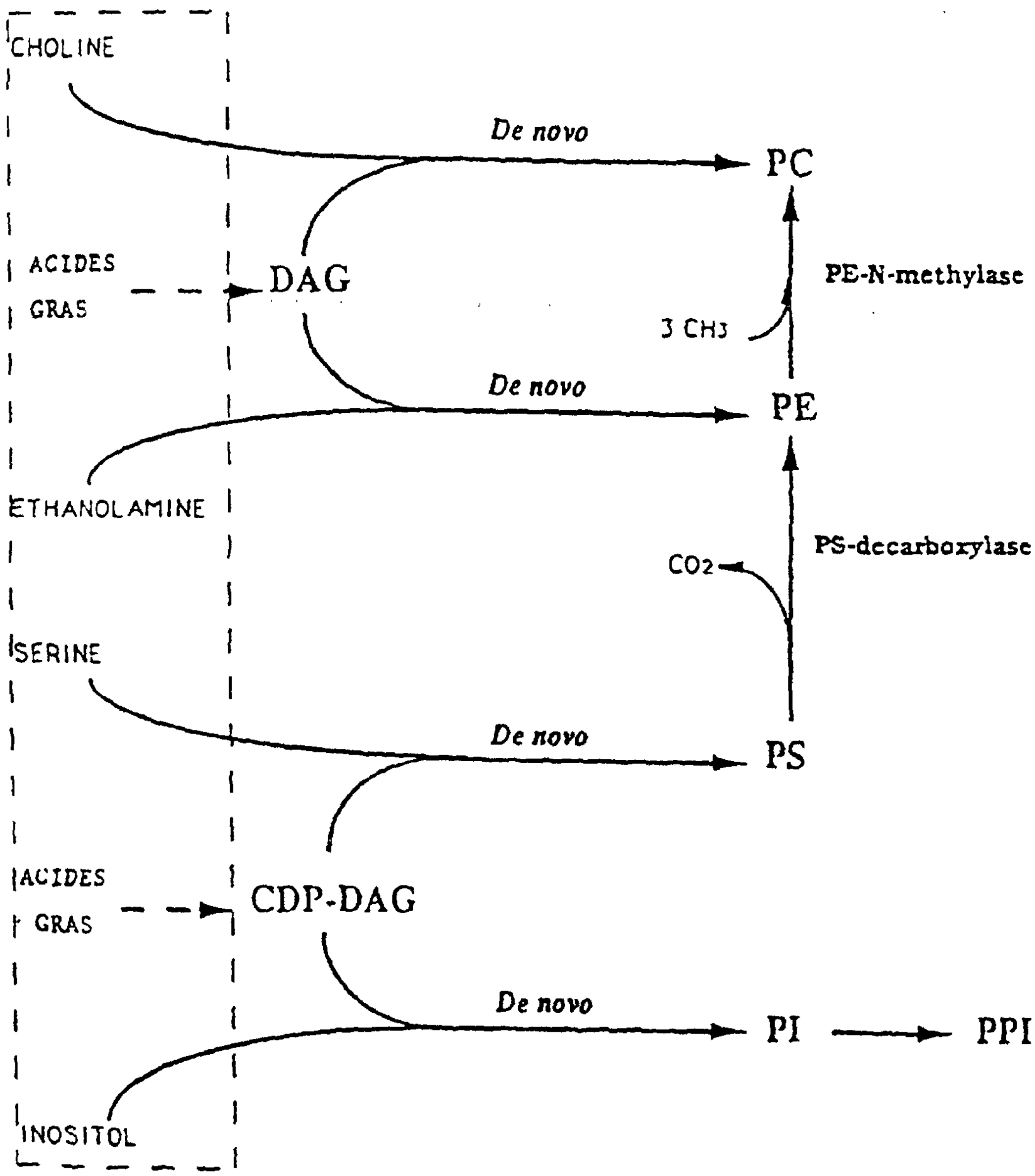
CHOLINE
De novo
PC
ACIDES
GRAS
DAG
PE-N-methylase
3 CH3
De novo
PE
ETHANOLAMINE
PS-decarboxylase
CO2
SERINE
De novo
PS
ACIDES
GRAS
CDP-DAG
De novo
PI
PPI
INOSITOL

Figure 1

Figure 2

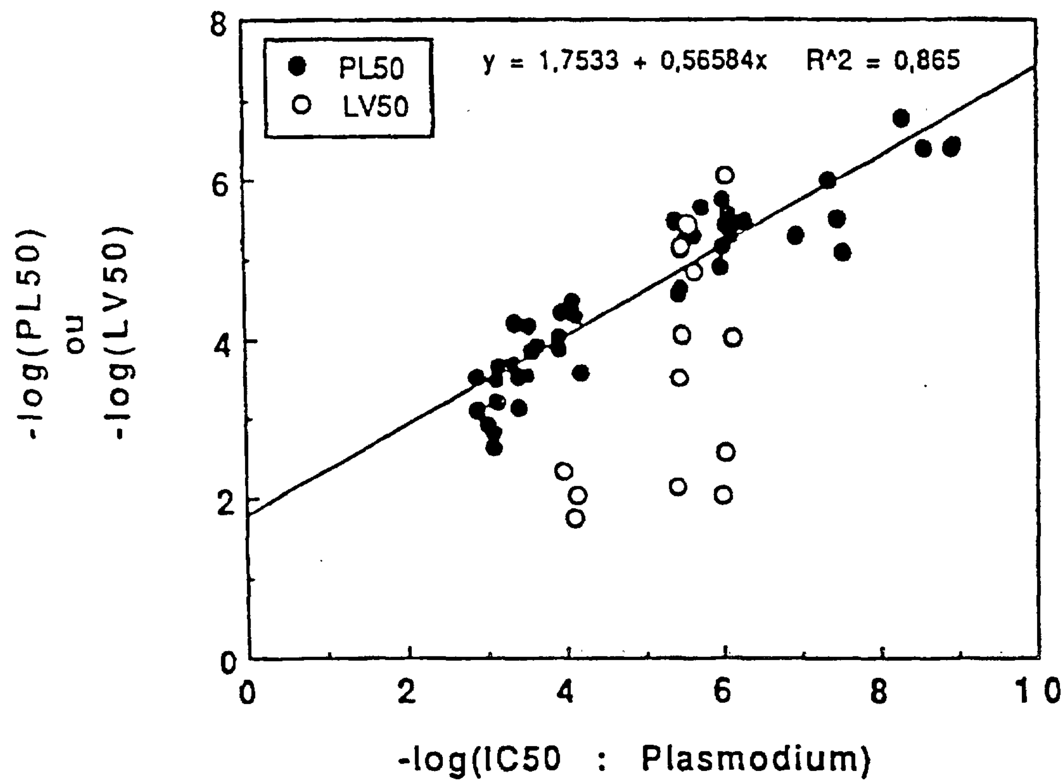
PL50
LV50
y = 1,7533 + 0,56584x R^2 = 0,865
-log(PL50) ou -log(LV50)
-log(IC50 : Plasmodium)
0
2
4
6
8
1 0

A:

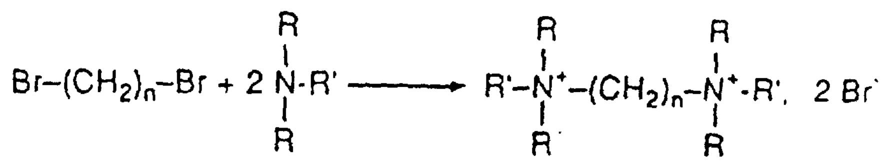

B:

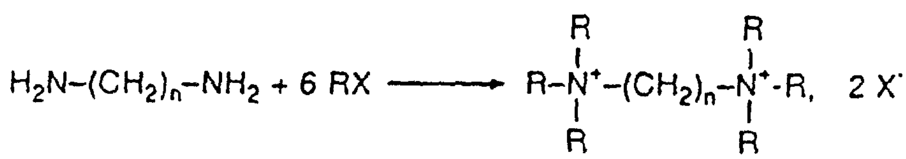

(X = Br ou I)

FIGURE 3

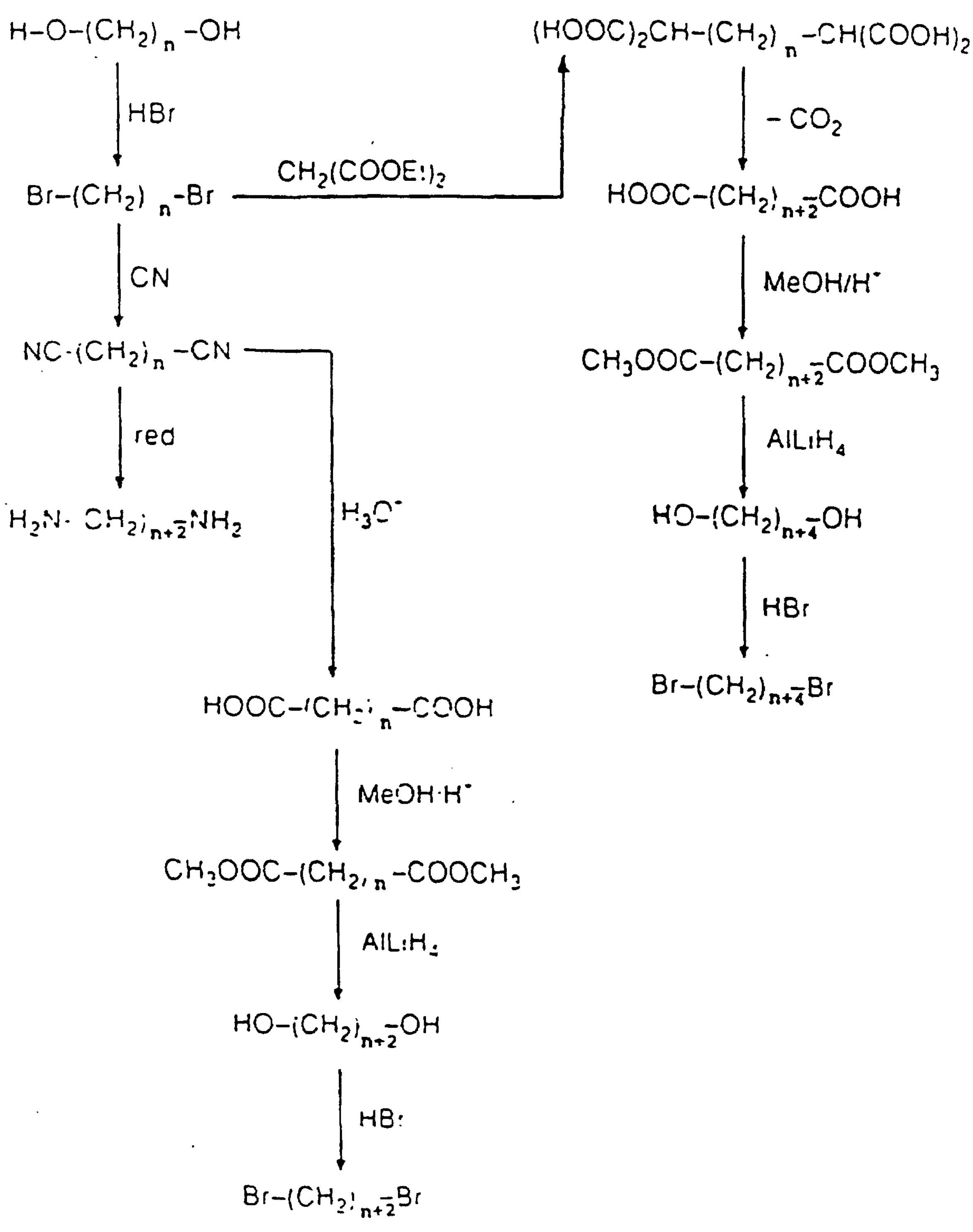
HBr
CH2(COOEt)2
CN
- CO2
MeOH/H+
red
H3O+
AlLiH4
HBr
MeOH·H+
AlLiH4
HBr

FIGURE 4

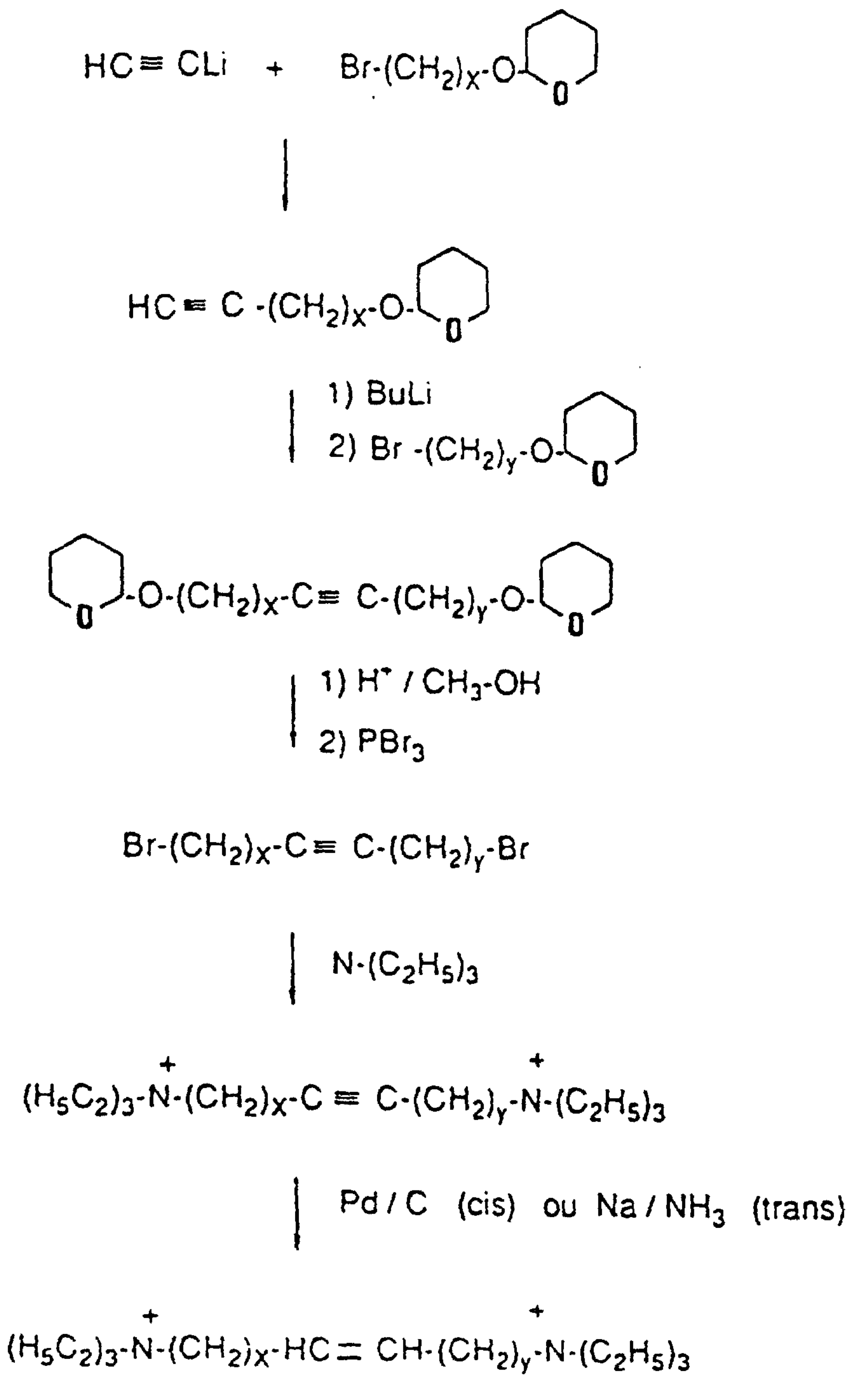


FIGURE 5

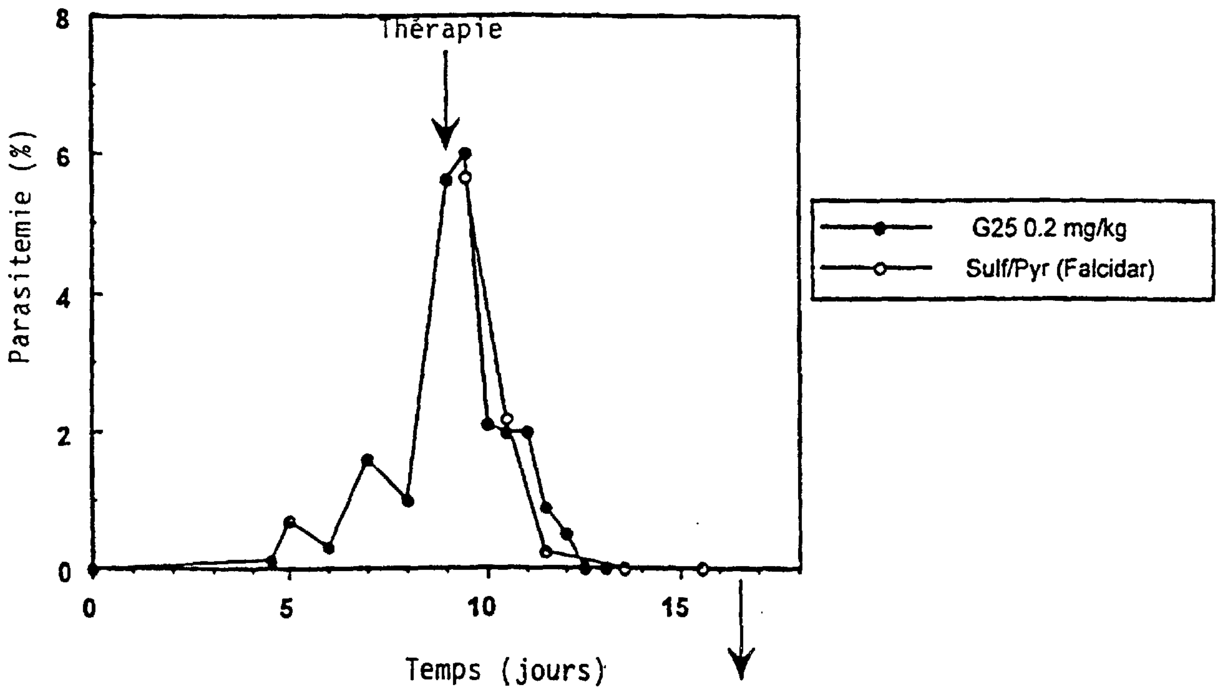
Thérapie
Parasitemie (%)
8
6
4
2
0
0
5
10
15
Temps (jours)
G25 0.2 mg/kg
Sulf/Pyr (Falcidar)

FIGURE 6